# EUROPEAN PATENT APPLICATION

(11) **EP 2 431 481 A1**
(43) Date of publication of application: **21.03.2012**
(21) Application number: 10075554.5
(22) Date of filing: 20.10.2004
(51) Int. Cl.: C12Q 1/68

(54) **Parallel polymer sequencing methods**

(30) Priority: 20.10.2003 GB 0324456
(62) Divisional of application: 04768958.3
(71) Applicant: Mir, Kalim, Oxford OX1 4LH (GB)
(72) Inventor: Mir, Kalim, Oxford OX1 4LH (GB)

(57) **Abstract**

The present invention relates to a method of sequencing a target polynucleotide by enzymatic and/or chemical means. The sequencing method includes a method for characterizing multiple alleles in a sample, a method for comparing polynucleotide sequences. The method involves analysing molecules as members of an array. Many target polynucleotides or many segments of a single target polynucleotide can be sequenced simultaneously. In a preferred embodiment the method involves analysing individual molecules within an array and base calls are based on the signals from two or more molecules. A method to prevent non-specific signal in sequencing is also provided. The invention is readily automated, both for small-scale and large-scale operation and relevant algorithms and the composition of kits and systems are provided.

## Description

The present invention relates to a method of sequencing a target polynucleotide by enzymatic and/or chemical means. The sequencing method includes a method for characterizing multiple alleles in a sample, a method of calculating confidence levels in ascertained sequences, a method for comparing polynucleotide sequences and a method of resolving ambiguities in a polynucleotide sequence. It also provides methods for appropriately preparing samples, for immobilising template molecules, for organising the template molecules and to conduct the sequencing of many molecules in parallel. The method involves analysing molecules as members of an array. Many target polynucleotides or many segments of a single target polynucleotide can be sequenced simultaneously. In a preferred embodiment the method involves analysing individual molecules within an array and base calls are based on the signals from two or more molecules. A method to prevent non-specific signal in sequencing is also provided. The invention is readily automated, both for small-scale and large-scale operation and relevant algorithms and the composition of kits and systems are provided.

The current methods of sequencing nucleotides are both time consuming and expensive. Sequencing the human genome for the first time took more than ten years and hundreds of millions of dollars. If re-sequencing of genomes and *de novo* sequencing of pathogens and model organisms could be performed several orders of magnitude faster and more cheaply it would open up new avenues for disease genetics and functional genomics.

Historically there have been two successful approaches to DNA sequence determination: the dideoxy chain termination method, e.g. Sanger et al, Proc. Natl. Acad. Sci., 74:5463-5467 (1977); and the chemical degradation method, e.g. Maxam et al, Proc. Natl. Acad. Sci., 74:560-564 (1977).

Sanger dideoxy sequencing which provides sequence information rather indirectly, by looking at the differences in gel-migration of a ladder of terminated extension reactions provided the means to sequence the consensus human genome. The chain termination method has been improved in several ways, and serves as the basis for all currently available automated DNA sequencing machines.

Now, however, the need for large scale re-sequencing of individual human genomes, *de novo* sequencing and re-sequencing in pathogens and model organisms require cheaper and faster alternatives to be developed.

The gel electrophoretic separation step, which is labor intensive, is difficult to automate, and introduces an extra degree of variability in the analysis of data, e.g. band broadening due to temperature effects, compressions due to secondary structure in the DNA sequencing fragments, inhomogeneities in the separation gel. Distinguishing the presence and identity of multiple sequences with current sequencing technology is virtually impossible, without additional work to isolate and perhaps clone the separate species of DNA.

Several methods that would avoid gel electrophoresis, cloning or the Polymerase-chain reaction (PCR) have been suggested. One ambitious approach is nanopore sequencing. However, this method does not reliably discriminate all 4 bases and the footprint of the nanopore is too large to achieve the required single base resolution. Despite initial optimism there has been little sign of progress towards sequencing directly by Atomic Force Microscopy (AFM) and Scanning Tunnel Microscopy (STM) and nor for that matter by Electron microscopy.

Methods are being explored in which the concept of determining sequence information by cleaving bases or by template directed synthesis is implemented in ways that avoid gels.

Sequencing by exonuclease digestion of individual nucleotide from single DNA molecules is one of the oldest of these approaches (CA1314247). However this method requires all bases to be contiguously fluorescently labelled which is difficult, and also there has been difficulty in preserving the order of the nucleotides between the time they are cleaved and detected.

A different cleavage based approach described in WO97/46704 involves ligation onto recessed 3' ends of sticky ended duplexes which contains the recognition site for a Type IIs nuclease, which cleave at a sites distal to the recognition site. Upon cleavage a few bases of the template are exposed, from which one base of sequence can be obtained for example by extension of a fluorescent ddNTP and ligated again with the sticky duplex containing the Type IIs recognition sequence. This can be iterated so that the strand is progressively sequenced as it shortens. This approach involves several enzymatic steps just to obtain one base of sequence information but it has the advantage that it can be conducted on double stranded DNA, which is the native form of DNA relevant to most applications.

The opposite approach of "sequencing by synthesis" (SbS) is described in US5302509, involves the identification of each nucleotide immediately following its incorporation by a polymerase into an extending DNA strand. One SbS approach, pyrosequencing, is widely used for SNP (single-nucleotide polymorphism) typing. In this case, the detection is based on pyrophosphate (PPi) release, its conversion to ATP, and the production of visible light by firefly luciferase. However, because the signal is diffusible, pyrosequencing cannot take advantage of the massive degree of parallelism that becomes available when surface immobilised reactions are analysed. It also adds only one nucleotide type at a time and this leads to a greater chance of misincorporation.

Therefore sequencing by synthesis in which all four nucleotides are added simultaneously and distinguishing them by labelling one or more of those nucleotides with different dyes would be preferable. However, the presence of dye molecules in contiguous bases leads to dye-dye interactions which cause fluorescence quenching. Also polymerases tend to "choke" on contiguous nucleotides that are modified with bulky groups. Moreover, if more than one dye labelled nucleotide is incorporated, as should be the case for a run of two or more of the same nucleotide, then it is difficult to quantitate the number of bases added; fluorescence signal intensity does not correlate linearly with the number of molecules.

US5302509 and Metzker et al (1994) disclose sequencing by synthesis strategies, which involve repetitive cycles and use reversible terminators to prevent the addition of more than one base at a time. In this method the nucleotide which is incorporated is modified so that it has a blocking group, which prevents the addition of further nucleotides, and a label. Once the incorporated nucleotide has been identified the blocking group is removed to allow the next nucleotide to be incorporated. The downside of this approach is that the chemistry used to remove the blocking group can damage the DNA and it is difficult to ensure that the deblocking reaction goes to completion. This means that the state of progress of different molecules of the population can become out of phase and at any given cycle one molecule in the population may be adding a different base to another molecule. Also if the label is at a distinct location other than the blocking group, it too needs to be removed.

WO96/27025 describes the sequencing by synthesis reactions and their analysis at the single molecule level.

Monitoring reactions on individual molecules rather than molecular ensembles enables longer read lengths to be obtained. Because each reaction is a stochastic process, the molecules within the array spot will extend asynchronously with respect to each other. Although, the stepwise addition of reagents limits the degree of phase difference between molecules, it is likely that at each cycle there will be a fraction of molecules in which either base addition or removal has failed. In the next cycle, addition or removal may resume on these molecules. The number of molecules that have dropped out of synchrony (i.e. missed addition/removal) will increase in the population as the synthesis progresses, and very quickly the point will be reached in which molecules that are out of synch, out-number those that remain in synchrony. Because of this asynchronous noise-i.e. a mixture of signals from different bases-the sequence signal is obscured and cannot continue to be obtained.

Exactly the same molecular process will occur when single molecules are being analysed but those molecules that have maintained synchrony throughout can still be followed even if they are a minority; because of the digital nature of the signal it cannot be swamped out by asynchronous molecules. Moreover the asynchronous molecules will also provide useful information when the data obtained from them is re-aligned in phase after the reaction. Finally, a stage will be reached where although synchronous information may continue to be obtained in a minority of the population, the number of molecules comprising this minority are not enough to continue to sequence with confidence. At this time the process can be reiterated on a new array based on the sequence information that has been obtained.

In contrast to some chemical approaches for the removal of blocking groups, the use of enzymes is gentle on the DNA and conditions can be manipulated so that reactions go to completion. US2003/194722 discloses the attachment of blocking groups via enzyme cleavable bonds, e.g. peptide bonds which can be cleaved by proteases.

One strategy removes an entire base rather than just a blocking group. US2003/013101 discloses a method of removing a base in its entirety, using the 3' to 5' exonuclease activity of a DNA polymerase. However, this method may remove more nucleotides than are added during a specific synthesis cycle, which can cause errors such as aberrant repetitions in the sequence.

One disadvantage of sequencing with fluorescently labelled nucleotides is non-specific adsorption to surfaces. This is particularly problematic when single molecules are analysed and the template molecules are immobilised on a surface.

Methods for polypeptide sequencing include the Sanger method. More recently mass spectrometry-based methods have evolved. However no methods exist or are known to have been proposed that allow highly parallel polypeptide sequencing.

The present invention provides new sequencing by synthesis methods to overcome the shortcomings of the methods disclosed in the prior art.

The present invention provides a method of sequencing a target polynucleotide comprising the steps of:
(a) Carrying out template derived/directed polynucleotide synthesis involving the incorporation of a labelled nucleotide (from the four types of nucleotides provided) ;
(b) detecting the presence or absence of said labelled nucleotide;
(c) replacing said labelled nucleotide with an unlabelled nucleotide; and
(d) repeating steps a) to c) so that the sequence of a template molecule is progressively obtained in the 3' or 5' direction;
   with the provisos that if said labelled nucleotide is labelled with a label directly attached to the nucleotide, then the replacement of said labelled nucleotide comprises removal of the entirety of a labelled nucleotide and replacement with an unlabelled nucleotide, and only said labelled nucleotide can be replaced.

In all aspects of the present invention wash steps may be introduced between each step as required. Particularly unreacted fluorescent nucleotides can be washed away before detection steps.

"Template derived polynucleotide synthesis" as used herein means forming a polynucleotide molecule utilising a polymerizing reagent that specifically incorporates nucleotides using the target nucleotide sequence as a template. The polymerizing reagent specifically incorporates nucleotides consistent with the well know Watson Crick base pairing rules to generate a complementary strand to the template The incorporation may be of nucleotide analogues, nucleotide mimics or other molecules which can be templated by a polynucleotide and in which pairing is by well defined rules (Eckardt *et al* 2002; Czlapinski, *et al,* 2001). For example, high-fidelity templating of DNA base shape mimics without forming Watson Crick Bonds has been reported (Delaney *et al,* 2003). Vice versa, the template may be any molecule which can template polynucleotide synthesis.

Polymerizing reagents include DNA polymerases, RNA polymerases, RNA transcriptases, reverse transcriptases, or ligases, as well as chemical reagents that enable template directed polymerization. As used herein "polymerising reagent" also includes molecules or complexes that are capable of enforcing high fidelity base pairing according to well defined rules, regardless of whether they catalyse the addition of a single nucleotide. They can be natural, such as those listed above, or artifical such as abzymes and ribozymes. The polymerizing reagent may comprise one or more chemical reagents. For example, template directed ligation can be mediated by chemical reactions (Xu *et al,* 2001; G. von Kiedrowski, 1986).

The target polynucleotide and synthesised polynucleotide can each independently be strands of RNA or DNA. The DNA can be genomic DNA, or cDNA. The RNA can be mRNA, or genomic RNA, such as that from a virus. Alternatively the target polynucleotide and/or synthesised polynucleotide can have an amide backbone formed through peptide nucleic acids (PNA) or a ribose P backbone, as formed by DNA.

The synthesis process can involve annealing a primer to the template polynucleotide. The primer can then be extended by template derived synthesis. The primer consists of 5-100 nucleotides, preferably 10-75, 15-65, 20 - 55, 25 - 50, or 30 - 45 nucleotides. The primer may be labelled. A primer may be made and then hybridised to the target polynucleotide. The primer may be composed of nucleotide analogues or mimics or any modification that improves its function as a primer. Alternatively nicks can be made in double stranded molecules using for example, Deoxyribonuclease 1 (DNAse 1) or Nickase optimised so that the distance between each nick is reasonably defined. The intact strand is the target polynucleotide to which a series of primers are annealed. Synthesis, and thus sequencing can start at each nick site and the non-template strand become progressively displaced. Whether a nick seeds displacement synthesis in a sense or antisense strand is revealed by the direction of migration of the sequencing signal. The strand and location of the nick is known when it is produced by an endonuclease that cleaves only one strand of its recognition site. The template polynucleotide is preferably attached or tethered to a solid surface.

The template can be attached indirectly to a surface or via a polymerizing reagent, which is attached to the surface, or it can be captured by a capture probe/primer. The capture may be of a single stranded target or a cohesive termini or "sticky end" of a double stranded template. The capture may also be of a double stranded region by, for example, RecA mediated strand exchange or blunt ended ligation.

RNA promoters native to the template DNA can be used for RNA synthesis by RNA polymerase. Alternatively, extrinsic promoters for specific polymerases can be incorporated by being part of a capture probe or by transposon directed integration into sites along the polynucleotide. T7 and T3 RNA polymerase promoters are preferred extrinsic promoters.

The term "labelled nucleotide" as used herein means any of the standard deoxyribonucleotides, or ribonucleotides which is attached to a label. Alternatively the nucleotides include any modified nucleotides or variations which pair with other bases according to defined rules, such as the Watson-Crick base pair rules. Thus the labelled nucleotide can be a labelled peptide nucleotide capable of forming PNA.

The term "labelled nucleotide" is not restricted to meaning a single nucleotide but in some embodiments of this invention the term includes a string of two or more nucleotide monomers, for example an oligonucleotide. An oligonucleotide consists of 3-30 nucleotides, preferably, 5-25, 10-20, or 12 -15 nucleotides. The oligonucleotide may be partially randomized and partially defined (see Ecker *et al*)*.* The defined portion provides the sequence information. The random portion stabilises the interaction with the template, provides an appropriate substrate for specific chemical or enzymatic reactions and provides sites for attachment of the label and terminator.

The label can be radioactive (such as ³²P), or more preferably a fluorescent tag. The fluorescent tag may be a dye molecule such as a fluorphore, for example the Cy dyes (Cy³ and Cy⁵), ROX (carboxy -x- rhodamine), TAMRA (tetramethylrhodamine), Oregon Green ®, Vistra Green ™, Fluorescein, PicoGreen ®, BODIPY ® series and Texas Red ®, the Alexa Dyes, the Atto Dyes, the Dyo dyes and the EVO dyes. Such fluorophores are commercially available, for example, from Atto-tech (Germany), Amersham (UK) or Molecular Probes (USA) (Kricka LJ.). Alternatively the label can be a tag which can be identified due to its physiochemical properties, e.g. electrophoretic mobility or an electric charge. Alternatively a raman signal can be detected, for example Surface Enhanced Resonant Raman Scattering (SERRs) (Kneipp 1999; Zander 2002).

Alternatively the nucleotide can be labelled with a mass tag and mass spectrometry could be used to read the identity of the added nucleotide.

The label can also be a nanoparticle, or microsphere. The nanoparticles may be optically active. For examples SERS particles, PRPs (Plasmon Resonant Particles), Quantum Dots, or latex particles with embedded dye, such as Fluospheres and Transfluospheres (Molecular Probes). The label can be a reporter and/or a terminator label. A reporter is a label that functions to report the identity of the nucleotide that is incorporated A terminator or blocker is a label that prevents the addition of more than one nucleotide until it is removed. In some cases fluorescence may be intrinsic to the nucleotide base; some base analogues have enhanced fluorescence. The fluorescence can be enhanced by proximity related effects with metals.

The label can be attached directly through a covalent bond to the nucleotide, or via a linkage. The linkage preferably comprises a cleavable bond, for example a photocleavable bond, or a bond which is cleavable by a mild chemical treatment, for example using a reducing agent to cleave a disulfide bridge. The linkage can preferably comprise a binding pair.

### Sequencing methods

The processing of sequencing relies on the base pairing that occurs between nucleotides to form a double stranded polynucleotide molecule, according to the Watson-Crick base paring rules. At each position in a nucleotide molecule, one of the four nucleotides can be incorporated. The nucleotide incorporated into the extending primer or into an RNA copy is normally the correct base that pairs with the base in the target polynucleotide

The sequencing method can be carried out in two ways. The four nucleotides can be labelled with the same label e.g. one fluorophore. The primer/template polynucleotides can be contacted with one nucleotide (e.g. adenine). The unincorporated nucleotides can then be removed either by being washed way or degraded or inactivated by enzymes such as Apyrase (Sigma chemicals) or Shrimp Alkaline Phosphatase (Amersham). Any nucleotides that have been incorporated can then be detected. This process can then be repeated with the other three nucleotides (e.g. thymine, cytosine and guanine). Alternatively, and preferably the four nucleotides can be differentially labelled i.e. each has a different label or fluorophore. In this case the primer and template polynucleotides are contacted with two or more of the labelled nucleotides at the same time. If required any free nucleotides are removed and incorporated bases are detected. The use of four differentially labelled nucleotides can allow continuous (real-time) monitoring of the synthesis process. The supply of all four nucleotides also reduces misincorporation. In one alternative embodiment sequencing may be of only two labelled bases and the other two bases are provided but are unlabelled. After sequence information is obtained of the first two bases the sequencing repeated with the other two bases labelled. The same can be done on the basis of 3 labelled nucleotides.

It is difficult to incorporate contiguous fluorescently labelled bases. One approach being explored is to adapt polymerases to incorporate non-native nucleotides. Another way to overcome this problem is to attach the dye via an appropriate linker. The chemical composition of the linker is chosen so that it minimally perturbs the polymerase function. The label is held at a distance significantly longer than is typically used (for example, a 12 atom linker is typical). This length may be greater than 12 nucleotides and may be between 13-150 atoms, 19-140 atoms, 36-130 atoms, 54-120 atoms, 72-110 atoms or 90-100 atoms. The fact that the linkers are at such distances provide a large degree of freedom and will contribute to the minimisation of dye-dye interactions and quenching.

Hence, sequencing will involve incorporation of bases at consecutive positions which are separated from their labels by extraordinary long linkers. The signal will increase linearly with the number of dyes incorporated because of the minimisation of dye-dye interactions and quenching effects. The signal may be bleached as required to detect subsequent incorporations more easily. Alternatively, the fluorophore and the label may be removed (e.g. by cleavage) or the fluorophore can be chemically modified to remove the fluorescence. The addition of Diphenyliodonium(DPI) chloride solution in the presence of photo-irradiation is able to destroy photoexcited fluorescein, irreversibly quenching the fluorescence (Aksyonov *et al* 2004).

Another way to overcome this problem is for fluorescent bases to be diluted by non-fluorescent bases. However, it is challenging to get this approach to work because enzymes prefer to incorporate the native nucleotides compared to the fluorescent nucleotides. One solution is to use only modified nucleotides. For each base (A, G, C, T) two modified nucleotides are present, both of which are incorporated with equal efficiency by the polymerase used. One modified nucleotide is labelled, and one is unlabelled. The labelled nucleotide is present at a lower concentration than the unlabelled nucleotide e.g. 10%. This will result in labels statistically not being incorporated at contiguous positions in a single molecule, but statistically will be incorporated in a fraction of the molecules, which is enough to be detected. As well as preventing the polymerase from choking, when there is a contiguous run of the same nucleotide in the sequence, this will also allow the number of nucleotides to be determined as the fluorescence intensity will increase discretely and will be linear, compared to the non-linear fluorescence intensities found when the labels are close together in the same molecule. This approach can be conducted by providing both versions of each of the nucleotides at a time. If all four bases are added (in label and non-labelled modified form) then the sequence could be monitored continuously. However because this is an ensemble approach there will be a tendency for the population of synthesis reactions to dephase, which would limit the read length. This can be overcome by capping any unblocked or unreacted chains so that they no longer participate in the reactions.

In both the above embodiments the fluorescence signal should be shortlived and able to photobleach whereas the incoming nucleotides are not liable to photobleach. This can be achieved by a Total Internal Reflection Fluorescence Microscope (TIRF) illumination system where only nucleotides at the surface are within illumination range to receive sufficient numbers of photons to photobleach. It can also be done by using nucleotides that bear a quencher as well as a fluorophore (this is discussed in detail in a later section).

In another embodiment, synthesis can be done in a stepwise manner, by only allowing the synthesis to increase by only a single nucleotide at a time.

This can be done by providing a block to nucleotide incorporation beyond a single nucleotide. This can be achieved by providing a removable blocker.

In one embodiment blockers and/or labels are removed but whole nucleotides are not removed (see nanoparticle section). In a preferred embodiment whole nucleotodies are removed within a single sequencing step. These methods are outlined in the following:
In one embodiment the label can be "directly attached" to the nucleotide via a covalent bond to the base, sugar moiety or alpha-phosphate.

Thus, in another aspect the present invention provides a method of sequencing a target polynucleotide comprising the steps of:
(a) extending a primer annealed to said target polynucleotide utilising a labelled nucleotide wherein the label is directly attached to the nucleotide;
(b) detecting the presence or absence of said labelled nucleotide within said extended primer;
(c) removal of said labelled nucleotide, and replacement of said labelled nucleotide with a degradation resistant nucleotide; and
(d) repeating steps a-c;
wherein the 3' end of said primer comprises at least one degradation resistant nucleotide.

When the nucleotide is attached through a direct covalent bond to the label, then the replacement of the labelled nucleotide with an unlabelled nucleotide comprises removing the entire labelled nucleotide, and replacing it with a degradation resistant unlabelled nucleotide. "Degradation resistant" nucleotides are nucleotides which are not removed from the synthesised nucleotide sequence by degradation agents. Conversely a "degradation labile" nucleotide is removed by degradation agents. "Degradation agents" as used herein refers to a reagent such as a chemical or enzyme that degrades a polynucleotide or it refers to a physical agent such as ultra-violet light.

A "degradation resistant bond" is an intranucleoside bond which is not cleaved by the presence of degradation agent, such as a phosphorothioate bond. Conversley a "degradation labile bond" is an intranucleoside bond which is cleaved in the presence of a degradation agents. Obviously some intranucleoside bonds may be resistant in the presence of one degradation agent, but labile in the presence of a different degradation agent.

The term "degradation" is used to include processive degradation from the 5' or 3' end or, cleavage of an internucleoside bond at any defined position.

In one embodiment this is preferably done by removing nucleotides from the 3' end. Such agents include exonucleases, such as exonuclease III, phosphodiesterases and includes DNA polymerases which possess 3' - 5' exonuclease activity. These enzymes include T4 polymerase, and *E.coli* DNA polymerase I (DNAPI). There are various types of degradation resistant nucleotides (Verma and Eckstein 1998). Preferably the degradation resistant nucleotides are α-thio-triphosphate (α-S-nucleotides). Polymerases only accept the Sp diastereomer and the invert the configuration to Rp upon incorporation, so that the resulting extension product can be degraded strong proofreading polymerases (Eckstein-F 1985). However, weakly proofreading polymerases have been identified which can be used (Di Giusto and King 2003). Even exonuclease III degradation has been shown to be truncated by these linkages (Lutz et al Nucleic Acids Res. 29(4):E16. 2001). Also the Rp diastereomer is more resistant than the Sp diastereomer to certain phosphodiesterases (Heaton and Eckstein 1996). The degradation resistant nucleotides may also be nucleotides which form methylphosphonate linkages or Locked Nucleic Acid (LNA). Also, for example boranophosphate modification at the alpha-phosphate group in 2'-deoxycytidine 5'-triphosphate (dCTP) (He *et al* 1999) may be used. Degradation reagents may also be chemical reagents such as mild reducing agent, a mild acid or alkaline. Physical degradation reagents include ultra-violet light for cleaving photocleavable bonds.

This embodiment is similar to that disclosed in US2003/013101 which uses exonuclease activity to remove a nucleotide in its entirety. In the present invention by using degradation resistant nucleotides the removal of more nucleotides than the last one added in a specific synthesis cycle is prevented. The incorporation of the degradation resistant nucleotide shifts the sequence register to the next position for the next cycle. The scheme also begins with primers that have degradation resistant nucleotides at their 3' end or when synthesis is initiated from a nick in double stranded DNA the first addition is of a degradation resistant nucleotide rather than a labelled nucleotide.

The labelled nucleotides that are incorporated may be dNTPs or ddNTPS. The disadvantage of adding dNTPS is there is no absolute certainty as to how many fluorescent bases become incorporated at each cycle. The disadvantage of labelled ddNTPS is that although only one labelled base will be added, after detection and removal of this base, the nucleotide which replaces it needs to allow incorporation of the next fluorescently labelled nucleotide and therefore cannot be blocked at the 3' end. However, this may allow multiple bases to be incorporated. Therefore it is possible that more than one base may be added. This would shift the register beyond the last base that has been sequenced. This can be overcome by manipulating conditions, reaction components and reaction times so that, statistically, there is only the chance of incorporating one nucleotide complementary to each single molecule template, in one cycle. A reaction can be done in which the nucleotides are added for a short burst followed by a chase with apyrase enzyme which degrades free nucleotides. Addition of a labelled nucleotide may involve a different enzyme to addition of a degradation resistant nucleotide. Alternatively, removable blocking groups, as described in any of the prior art can be added to the 3' end of the unlabelled nucleotides. These can be removed before addition of the next labelled base. These blocking groups may be groups which substitute for the OH position on the 3' position of the sugar ring. Also labels attached to the DNA bases via S-S bonds may act as terminators.

One preferred embodiment, as shown in figure 4, involves the addition of a labelled ddNTP. This is removed in its entirety by a degradation reagent and replaced with a degradation resistant nucleotide which is also a ddNTP and hence only one nucleotide can be incorporated. The blocking of the ddNTP is then removed before repeating the cycle. The degradation resistant nucleotide may be a α-S-ddNTPs (TriLink, USA) and the degradation reagent may be an exonuclease activity of a DNA polymerase or an exonuclease. Exonuclease III is able to repair the aberrant dideoxy part of the nucleotide so that extension can continue. As an alternative to repair of the 3' end by exonuclease III, the nucleotide may be blocked from extension by a 3' phosphate. This can then be repaired to OH by Polynucleotide Kinases, making the end competent for extension. It can also be repaired by a 3' to 5' exonuclease in the presence of nucleotides.

In addition to blocking by the substitution of OH by H at the 3' of the nucleotide, it may be blocked by any type of other blocking group, for example a photocleavable 2-nitrobenzyl blocking group.

Primers with phosphorothioate linkages are available cheaply from most oligo synthesis houses. The Sp diastereomer of the primer can be prepared by preincubation with strong exonuclease activity so that the Rp diastereomer is removed. Extending a primer annealed to a target polynucleotide also includes extension from a nick in the DNA but in this case the first step must be incorporation of a α-S-nucleotide.

Alternatively instead of using blocked fluorescent nucleotides or blocked degradation resistant nucleotides, the incorporation of more than one nucleotide can be allowed to occur. The number of fluorescent bases added can then be deduced from the brightness of the signal or the photobleaching characteristics. Then, as long as the degradation resistant nucleotides are added with a similar efficiency, registration will not be lost in most instances. It should be noted that fluorescent signal may not increase linearly with the number of incorporated bases but instead may decrease. This is thought to be due to the quenching of adjacent fluorophores. However this problem may be avoided with the use of labels such as name particles which are also terminators (see nanoparticles section below).

Alternatively, although the degradation resistant nucleotide is not chemically blocked, the polymerizing reagent used to add the degradation resistant nucleotide may not be able to incorporate more than one degradation resistant nucleotides. However, the polymerizing reagent used for addition of the next labelled degradation labile nucleotide is able to add on to this base. Different enzymes have different processivities and different capacities to deal with natural and modified DNA nucleotides. The different steps may utilise different degradation reagents and different polymerization reagents. It should be noted that that although a frequent changing of polymerizing reagent is expensive, it is justifiable if sequencing is done on a large number of molecules in parallel. If the reactions are done in microfluidic channels the amount of reagents will be small and if a system of valves is incorporated onto a sequencing chip, the reagents which will usually be provided in excess amounts can be stored in designated chambers on the chip and re-used.

Also, to prevent addition of more than one nucleotide at a time both the degradation resistant and degradation labile nucleotide may be modified or the reaction configured to prevent the addition of more than one nucleotide during one cycle. In one preferred embodiment, a blocking group is added to the 3' position on the degradation labile nucleotide, restricting polymerization to a single base addition. The degradation reagent is able to remove the modified degradation labile nucleotide. After cleavage of this nucleotide by the degradation reagent, the degradation resistant nucleotide that replaces it also contains a blocking group at the 3' position, again restricting polymerization to a single base addition. This ensures that the sequence register is shifted by the required single position only. Although further degradation resistant nucleotides are not able to react at this 3' position, the degradation labile nucleotide is able to react at this position. Hence the process can continue. For example the degradation labile nucleotide may have an NH₂ group at the 5' position which will have different reactivity than a 5' phosphate on the degradation resistant nucleotide.

### Cleavage of an internucleoside P3'-N5' Phosphoramidate (P-N) linkage

In another preferred embodiment, as shown in figure 8, the labelled nucleotide is a degradation labile nucleotide. This can be a nucleotide modified, for example at the 5' position with NH₂ (Wolfe 2003; Shchepinov 2001) which can be efficiently incorporated into DNA by the Klenow fragment of *Escherichia coli* DNA polymerase. An example of such a ncleotide is a phosphoramidate nucleotide, e.g. NH2-dNTP, NH2-NTP or NH2-ddNTP. The resulting modified internucleoside bond can be specifically cleaved by chemical treatment such as mild acid treatment. In this case the degradation resistant nucleotide can be a normal nucleotide.

This embodiment can be carried out during either RNA (Gueroui 2002) or DNA synthesis,. Following detection, the labelled degradation labile nucleotide is replaced by a degradation resistant nucleotide in order to shift the register to the next position in the sequence. This approach can be carried out by primer mediated DNA synthesis or promoter mediated RNA synthesis. Details of synthesis of NH₂ nucleotides is provided by Wolfe *et al* (2003). The nucleotides can be labelled by standard methods (e.g. see Hermanson, GT or Mitra 2003). Custom nucleotides and labels attached thereon can be provided by several vendors including Jena-Bioscience, Perkin Elmer, Amersham, Fidelity systems.

The replacement of a labelled nucleotide with a regular or degradation resitant nucleotide may involve repairing 3' end of nucleotide. The labels that are used to label each of the four bases are distinguishable from each other, if one or more nucleotide is used at one time. When a labelled phosphoramidate nucleotide is a blocked at the 3' end, the chain can be extended by one nucleotide only. The internucleoside bond connecting the non-labelled nucleotide to the labelled nucleotide is labile to the chemical treatment. It is preferably blocked at the 3' end so that the chain can be extended by one nucleotide only and this blocking is removed prior to reiterating steps a-c.

The chemical treatment is preferably mild. For example, the phosphoramidate bonds formed within the resulting polynucleotides can be specifically cleaved with dilute acetic acid, for example 0.1M. In some instances measures are required to ensure that the extending primer remains complexed to the template after mild acid treatment. For example the primer may be covalently linked to the template or both primer and template may be linked to a surface, in intimate contact with each other.

The repair of the 3' end may be performed by a polynucleotide kinase. It can also be carried out by an exonucelase in the presence of nucleotides.

In another embodiment the label is attached to the nucleotide by means of a linker which comprises a cleavable bond, as described above. In the method of the invention, the replacement of said labelled nucleotide with an unlabelled nucleotide includes the removal of the label from said nucleotide.

The cleavable bond can be cleaved following the detection of the presence or absence of the labelled nucleotide. The label can be attached in such a way that it blocks the incorporation of further nucleotides. This ensures that the only one labelled nucleotide is incorporated. Thus the cleavable label may have dual functionality, blocking the addition of more than one nucleotide, as well as reporting the identity of the nucleotide.

The cleavable bond can be cleaved by means of light (if it is photocleavable). A photocleavable 2-nitrobenzyl linker at 3' end as a photoreversible blocker/label has been described by Li *et al* (2003). If the cleavable bond is a disulphide bridge it can be cleaved using a mild reducing agent such as 2-Mercaptoethanol, (dithiothrietol) dithiothrietol (DTT) and Tris(2-carboxyethyl)phosphine hydrochloride TCEP. If the removable bond comprises a diol then it can be selectively cleaved using saturated aqueous NaIO₄.

The label may not be directly attached to the linker but it may be attached via a linker to biotin or an analogue thereof, to which a streptavadin conjugated label is attached, for example, Atto-565-streptavidin (Sigma).

The intense affinity of biotin-binding proteins for biotin is essentially irreversible. Harsh treatment, extremely low pH and highly concentrated chaotropic reagents are required to break the association. As an alternative mechanism for providing an analogue biotin from which streptavidin can be easily decoupled can be used. 2-Iminobiotin (IBA, Gottingen, Germany) or Desthiobiotin (Glen research) whose association with biotin-binding proteins can be broken at pH4 or by elution with free biotin, can be used. Preferably these are connected to the nucleotide by a long linker.

Thus in one aspect the present invention provides a method of sequencing a target polynucleotide comprising the steps:
(a) Carrying out template derived nucleotide synthesis utilising a labelled nucleotide wherein the label is attached to the nucleotide via a cleavable linkage;
(b) detecting the presence or absence of said labelled nucleotide within the synthesised polynucleotide;
(c) cleaving said label from said nucleotide; and
(d) repeating steps a-c.

### Binding pair

In one preferred embodiment, the linkage attaching the label to the nucleotide comprises a binding pair. This is shown in figure 3. One member of the binding pair is linked to the nucleotide, preferably via a cleavable bond as described above. The other member of the binding pair is attached to the label such as a fluorescent dye or nanoparticle. A binding pair consists of two molecules, preferably proteins, which specifically bind to one another. The members of a binding pair may be naturally derived or wholly or partially synthetically produced. One member of the pair of molecules has an area on its surface, which may be a protrusion or a cavity, which specifically binds to and is therefore complementary to a particular spatial and polar organisation of the other member of the pair of molecules. Thus, the members of the pair have the property of binding specifically to each other. Examples of types of binding pairs are antigen-antibody, biotin-avidin, hormone-hormone receptor, receptor-ligand, enzyme-substrate. The use of a linkage comprising a binding pair means that the nucleotide added onto the primer may be labelled after it has been incorporated into the primer. The nucleotide is attached, preferably via a cleavable linker to one member of a binding pair. The label is attached to the other member of the binding pair. The label can then be attached indirectly to the nucleotide as the two members of the binding pair bind one another.

Each of the four types of nucleotides can be attached to a different binding pair member. The other members of the binding pair can be labelled differentially, i.e. each is attached to a different fluorophore or nanoparticle. This allows all of the nucleotides to be added at the same time. The nucleotide incorporated is then labelled with the respective fluorophore via the binding pair mechanism. For example adenine is attached to biotin, and cytosine is attached to digoxigenin. The fluorophore indicating the presence of adenine is attached to avidin, and that for cytosine is attached to anti-digoxigenin.

Thus in one aspect the present invention provides a method of sequencing a target polynucleotide comprising the steps of;
(a) carrying out template derived nucleotide synthesis using a nucleotide attached by a removable linkage to one member of a binding pair;
(b) contacting said nucleotide with a label attached to the other member of the binding pair under conditions such that the two members of the binding pair bind to one another;
(c) detecting the presence or absence or said label;
(d) removal of said label and said binding pair by removing said removable linkage between the first and second members of the binding pair; and
(e) repeating steps a-d.

### Quencher Sequencing

The nucleotides may be in a non-fluorescent state, for example a quenched state, until they are incorporated. This overcomes the problem of non-specific signal from unincorporated nucleotides, particularly those that stick to the slide or chip surface. This opens the way for using various types of simple slide surface chemistries as opposed to the complex polyelectrolyte multilayer treatments described by Kartalov *et al.* In addition, the combination of surfaces with low adsorption of fluorescent nucleotides with quencher nucleotides may be especially advantageous.

In WO00/36151 a quencher moiety is covalently bound to a nucleotide base and the fluorescent label is attached to the gamma phosphate. The reason for this is so that the fluorophore is released at each incorporation and the sequencing reaction can be monitored in real-time. However the aim of the present invention is different as it ensures that the only fluorophores that are detected are those which are incorporated. In addition, the fluorescence signal is resident at the location where the nucleotide is incorporated for an extended period of time. Particularly, the signal should be long-lived enough so that all four labels can be viewed and that different regions of the array can be imaged. This may require several exposures of a CCD camera at several 10s, 100s or 1000s of locations. This would not be possible with the short detection time afforded by a fluorophore that becomes released after incorporation.

Therefore, in a preferred embodiment of the present invention the labled nucleotide is attached to a quencher at the gamma position, and said fluorescent tag is attached at the 3' position or to the base. This embodiment is illustrated in figure 5.

After incoporation the quencher moity is removed and allowed to diffuse away and the fluorophore is able to fluoresce. In some ways this strategy has similarities to the TAQMAN™ 5' nuclease assay (Livak KJ 2003).

As used herein, a quencher is a moiety which decreases the fluorescence emitted by the fluorescent label. This includes complete, and partial inhibition of the emission of the fluorescence. The degree of inhibition is not important as long as a change in fluorescence can be detected once the quencher is removed.

The quencher moiety may be any quencher moiety and can selected from the group consisting of DABCYL, rhodamine, tetramethyl rhodamine, pyrene butyrate, eosine nitrotyrosine, ethidium, fluorescein, Malachite green, Texas Red, dinitrobenzene and trinitrobenzene. It may also be a nanoparticle. The quencher can be attached to gamma phosphate via a linker.

The fluoroescent label is any fluoroescent label that is capable of being quenched which includes the fluoroescent label, such as fluorophores mentioned elsewhere in this document. The fluorescence that is quenched may also emanate from a nanoparticle. The fluorescent label or flurophore and quencher moiety may interact via a mechanism selected from the group consisting of fluorescence resonance energy transfer, an electron transfer quenching mechanism and a ground-state complex quenching mechanism.

Also, the fluorescent label and quencher may be selected from the group consisting of fluorophores, quenchers, shift reagents, spin labels, radioisotopes, and magnetic reasonance contrast agents.

Also the fluorescent label may be selected from the group consisting of optionally substituted pyrenes, anthracenes, naphthalenes, acridines, stilbenes, indoles, benzindoles, oxazoles, benzoxazoles, thiazoles, benzothiazoles, 4-amino-7-nitrobenz-2-oxa-1,3-diazoles, cyanines, carbocyanines, carbostyryls, porphyrins, salicylates, anthranilates, azulenes, perylenes, pyridines, quinolines, coumarins, polyazaindacenes, xanthenes, oxazines, benzoxazines, carbazines, phenalenones, benzphenalenones, carbazines, oxazines, 4-bora-3a,4a-diaza-s-indacenes, fluorophoresceins, rhodamines, rhodols, 5-carboxyfluorophoresceins (FAM), 5-(2'-aminoethyl) aminonapthalene-1-sulfonic acids (EDANS), anthranilamides, terbium chelates, Reactive Red 4, Texas reds, ATTO dyes, EVO Dyes, DYO Dyes, Alexa dyes and BODIPY dyes.

Also the quenching moiety is selected from the group consisting of optionally substituted phenyls, naphthyls, anthracenyls, benzothiazoles, benzoxazoles, or benzimidazoles, pyrenes, anthracenes, naphthalenes, acridines, stilbenes, indoles, benzindoles, oxazoles, benzoxazoles, thiazoles, benzothiazoles, 4-amino-7-nitrobenz-2-oxa-1,3-diazoles, cyanines, carbocyanines, carbostyryls, porphyrins, salicylates, anthranilates, azulenes, perylenes, pyridines, quinolines, coumarins, polyazaindacenes, xanthenes, oxazines, benzoxazines, carbazines, phenalenones, benzphenalenones, carbazines, oxazines, 4-bora-3a,4a-diaza-s-indacenes, fluorophoresceins, rhodamines, rhodols, 5-carboxyfluorophoresceins (FAM), 5-(2'-aminoethyl) aminonapthalene-1-sulfonic acids (EDANS), anthranilamides, terbium chelates, Reactive Red 4, dabcyls, nitrotyrosines, malachite greens, Texas reds, dinitrobenzenes, ATTO dyes, EVO Dyes, DYO Dyes, Alexa dyes and BODIPY dyes.

DABCYL is a preferred quencher. Although the term quenching is used here, instead of a quencher there may be a second dye attached and the first and second dyes may interact as FRET partners as donors and acceptors or electron transfer donors and acceptors (the acceptor could also be nucleotide base such as Guanine in this case)-WO03/089670 describes Internally Quenched Nucleotide fluorescent reporters (IQNs) which have recently been introduced by Lawler Scientific/ Glen Research for incorporation into real-time PCR, microarray technologies and diagostics. In the present invention it is proposed that these reagents can be adapted for use in sequencing reactions.. Fluorescein-dUTP-dabsyl can be incorporated well by reverse transcriptases. This fluorophore quencher pair is well described in the literature (Marras *et al* 2002) Fluorescence emission is >98% quenched. Certain IQNs may be incorporated by DNA polymerases and thermostable varieties thereof. One IQN which can be used by thermostable polymerases is Pyrrolo-dCTP-dabcyl. Pyrillo is an instrinsically fluorescent nucleobase. US2004014096 also describes dual labeled nucleotides with quencher and fluorophore attached In addition, the following types of quenched nucleotides are available as a custom synthesis from Jenabioscience: (i) Fluorophore-5-Aminopropargyl-ddCTP-gammahexylamino-quencher ; (ii) Fluorophore-5-Aminoallyl-ddUTP-gammahexylamino-quencher; (ii) fluorophore-7-Aminopropargyl-7-Deaza-ddATP-gammahexylamino-quencher; (iv) fluorophore-7-Aminopropargyl-7-Deaza-ddGTP-gammahexylamino-quencher.

The same quencher structure can be provided at the gamma phosphate position of all four nucleotides, each bearing a distinguishable fluorescent label. Alternatively, different quencher structures can be provided for each of the four nucleotides bearing different fluorescent labels.

In addition to the gamma phosphate modification the nucleotides may have one or more phosphates replaced with phosphorothioate or phosphoramidate. For example the nucleotide may be NH₂-nucelotide or an α-S-nucleotide. The nucleotide may also be blocked at the 3' end and may be a dideoxynucleotide.

### Photo-clocking of sequencing

In an alternative embodiment to stepwise sequencing, quenched nucleotides can be used for sequencing by continuous or real-time monitoring of synthesis. However, as opposed to the prior art where the fluorophore resides at the gamma phosphate position and is released upon incorporation, in the present invention, the quencher at the gamma phosphate position released and the fluorophore remains on the nucleotide. The reaction conditions can be manipulated such that the fluorophore can then be photobleached or chemically inactivated before the next nucleotide has chance to be incorporated. The quenched nucleotides, because they are quenched will not be photobleached.

In one preferred embodiment, the fluorophore is attached on the via a photocleavable bond such as 2-nitrobenzyl which has a high photocleavage efficiency by UV light. If this is attached at the 3' sugar then it acts as a reversible terminator. Also depending on the nature of the structure, it may also be attached at the nucleotide base and function as a reversible terminator (Shendure et al 2004; Hennig. C AnyBase.nucleotides. GenoVoxx [online] http://www.genovoxx.de). In addition the bond may be acid cleavable, such as a PN bond and photogeneratable acids (*Gao et al* 1998; Gao *et al* 2001) are used. In another embodiment, acids can be generated in a solution of electrolyte by an electrode to which current is applied (Egeland *et al* 2002)).

In this scenario, the nucleotide is quenched until it is incorporated and only one nucleotide is capable of being incorporated in one cycle. The fluorescence from the incorporated labelled nucelotide remains detectable (subject to photobleaching, which can be attenuated by provision of antioxidants) for a required period, before it is removed directly or indirectly by the action of light or a current applied to an electrode. Once the fluorescent terminator is removed, the next nucleotide can be incorporated. Hence this can be operated as a closed system, where the reagents required for the reaction are provided at the start, and the sequencing cycles are iterated or clocked by the action of physical signals.

Alternatively the fluorescent tag may comprise a nanoparticle.

### Nanoparticle sequencing

The term "nanoparticle" as used herein means an individual particle which has a maximum dimension in any one direction of less than a micron. The nanoparticles of use in the present invention are preferably spherical, and/or preferably have a diameter of 20nm or less.

The fluorescent nanoparticle can be a particle which has a large number of fluorophores embedded within or on its surface (e.g. latex particles). Alternatively, fluorescence emission or modulation may be an inherent property of the particle as is the case for semiconductor nanocrystals (Quantum Dot Corp., USA; Evident Technologies, USA), gold nanoparticles (Nanoprobes Inc., USA), plasmon resonant particles (PRPs) (Seashell Technologies, USA), Resonance light-scattering particles (RLP) or TiO₂ nanoparticles (Paunesku 2003). Depending upon their size and/or material composition, semiconductor nanocrystals emit in different regions of the electromagnetic spectrum, even when excited with the same wavelength. Special coating procedures are applied to stabilize them in solution and make possible their conjugation with different objects. The advantage of nanocrystals is their high brightness of emission, high stability against photobleaching and their narrow emmision spectrums, which facilitates multi-plexing. Semiconductor nanocrystals, of various emission wavelengths, with surfaces coated with streptavidin or biotin are available form Quantum Dot Corp. The streptavidin-biotin interaction can be mediated in the vendor supplied incubation buffer or other commonly used buffers.

Nanoparticles can also be attached to a modified nucleotide via a thiol (sulfhydryl/mercaptan) group. Thiol groups can be attached to metals, in particular, gold. Alternatively, a linker can be used to attach the thiol to the nucleotide. The linker may contain a cleavable bond which is photocleavable or cleavable by a mild reducing agent. Several thiol moieties may branch off from a single nucleotide thus increasing the strength of interaction with the nanoparticle. Alternatively the nucleotide base may be labelled with an amino-allyl group.

When semiconductor nanocrystals are used, a Native CdSe/ZnS core/shell nanaocrystal is first capped with 3-mercaptopropionic acid. The carboxylic acid group is then deprotonated with 4-(dimethylamino)pyridine, rendering the nanocrystal water soluble, and facilitating reaction with thiolated nucleotides. After DNA modification, the particles are separated from unreacted DNA by dialysis, ultracentrifugation or gel electrophoresis. Solublised nanocrystals, emitting at various wavelengths are commercially available e.g. from Evident Technologies. Under the relatively mild reaction conditions used for enzymatic extension there is no appreciable thiol exchange.

Amine coated Quantum Dots are available form Quantum Dots Corp. (USA) and a kit is provided for linking them with thiol bearing biomolecules.

As an alternative to linking the nucleotide to a nanoparticle through a thiol group, the binding pair linkage system described above can be used. The nucleotides can be attached to one member of a binding pair (e.g. biotin) through a cleavable linker and the nanoparticle may be coated with the other half of the binding pair, e.g. streptavidin. A photocleavable-Biotin-NHS reagent is commercially available from AmberGen which can react with amine groups in the nucleotides. A SNHS-SS-BIOTIN is available from Uptima and Pierce Biotechnology (EZ-Link Sulfo-NHS-SS-Biotin) which can be attached to amines on DNA bases and is cleavable by a mild reducing agent. In addition Photoprobe biotin reagent is available from Vector laboratories which allows biotin to be linked to DNA by heat or UV exposure, with the option of a cleavable disulphide bridge within the linkage. Furthermore, Biotin linked to all four DNA nucleotides via a SS bond are available from as a special order (Perkin Elmer, USA)

Nanoparticles used in this invention are seen as a diffraction limited point source of fluorescence. The advantage over other single molecule sequencing by synthesis approaches is that the nanoparticle is much easier to detect than a single dye molecule. Therefore a low grade CCD camera can be used for their detection and illumination may be from a mercury arc lamp. Lower grade objective lenses may be used and oil immersion lenses are not necessary. Sophisticated set-ups for background elimination, such as evanescent wave illumination are not typically needed. Hence, the detection device is less sophisticated and cheaper than the instrument required for single dye molecule detection. Because a nanoparticle is easily distinguished from artefacts and over background, sample preparation is easier and less stringent. An instrument for detection of nanoparticles is available from Nanosight Inc.

Nanoparticles can be easily removed after detection. A gold or CdSe/ZnS Quantum dot can be removed by treatment with a mild reducing agent such as DTT or mercaptoethanol. The Au-S bond, although thermodynamically stable, is kinetically labile, leading to thiol exchange in the presence of appropriate thiol-containing molecules in solution, particularly at elevated temperatures. It is also possible to attach DNA to nanoparticles via a binding pair as described above, which would avoid the use of thiols altogether. The linker connecting the base to one of the binding pairs or the thiol may contain a cleavable bond such as a disulphide bond which can be removed using a mild reducing reagent.

The nanoparticle may bear a positive charge which can interact with the nucleotide (Nakao 2003). The charged nanoparticle can be displaced by another charged species, following detection.

Non-specific binding to surfaces can be reduced by treatment of surfaces with BSA or Caesin. Unlabelled nucleotides and various types of nucleic acids, such as yeast tRNA and salmon sperm DNA can be used for blocking surface. There are various commercial surface blockers available. It can also be achieved, particularly with certain nanoparticles by surface treatment with CsCl or MgCl₂

There are two specific ways that nanoparticles can be used in the invention to achieve sequencing by synthesis. The first involves addition of nanoparticle in order to label a base after it has been incorporated.

Thus in one embodiment the synthesis involves incorporation of a labelled nucleotide comprising:-
a) Incorporation of an unlabelled nucleotide adapted for the attachment of a nanoparticle; and
b) Labelling said unlabelled nucleotide with a nanoparticle

The unlabelled nucleotide can be adapted for the attachment of a nanoparticle by the presence of a thiol group or a member of a binding pair as described above.

The second method by which nanoparticles can be used involves incorporation of nucleotides to which nanoparticles are already attached.

Thus in another embodiment the replacement of the labelled nucleotide, which is labelled by means of a nanoparticle, with an unlabelled nucleotide comprises cleaving the nanoparticle from said labelled nucleotide. The nanoparticle can be removed by cleaving the cleavable bond attaching the nanoparticle to the nucleotide. The cleavable bond may be cleaved by light if it is photocleavable, or by means of a mild reducing agent, such as mercaptoethanol or DTT, if it is a disulphide bond.

The use of nanoparticles means that additions of multiple nucleotides can be detected more easily because the increase in signal is not quenched and so a digital increase in signal intensity can be expected with increasing number of nucleotides.

WO96/27025 discloses labelling nucelotides with microscopic beads in the context of sequencing by synthesis. The reagents used in the present invention are specifically nanoparticles of 20nm diameter and less as significantly larger beads would be too bulky to efficiently carry out the required molecular processes.

The nanoparticle strategy of the present invention differs from WO96/27025 in that the nanoparticles are not only used for labelling but may also serve to prevent the incorporation of more than one nucleotide per cycle. The addition of a second base may be prevented by steric hindrance or repulsion. The nanoparticles may have a polarity, which repels another. For example they may be positively or negatively charged (Nakao 2003) or they may have a magnetic polarity or spin (Lee 2003).

In addition to detection due to fluorescence, nanoparticles can also be detected efficiently by electron microscopies or scanning probe microscopies (e.g. see Csaki 2001)

### Sequencing by ligation

Another embodiment of the invention the labelled nucleotide is an oligonucleotide and step (a) comprises ligating said oligonucleotide to a primer annealed to said target polynucleotide.

This method does not involve new chemical modifications, but retains the use of exonuclease and degradation resistant/labile linkages without allowing more than one nucleotide to be added at each cycle. Although the strategy is based on template directed synthesis, instead of polymerisation of single nucleotides by DNA polymerase, the nucleotide incorporated can be at one end of an oligonucleotide. The label is attached to one of the other nucleotides within the oligonucleotide. The polymerisation occurs by ligation of oligonucleotides to a growing chain in either the 3'to 5' direction or the 5' to 3' direction. The nucleotide to be incoporated into the synthesised polynucleotide forms a base pair with corresponding base in the template polynucleotide.

In one embodiment the polymerizing agent joins the oligonucleotide to the polynucleotide being synthesised utilising a degradation resistant bond, for example a methylphosphonate or phosphorothioate linkage. The polymerizing agent may consist of a DNA ligase or a set of chemical ligation reagents. Chemical ligation methods are well known in the art, e.g. Ferris et al, Nucleosides & Nucleotides, 8:407-4.14 (1989); Shabarova et al, Nucleic Acids Research, 19:4247-4251 (1991).

The presence of the incoporated nucleotide is detected utilising the label attached to another nucleotide in the oligonucleotide. Once the signal has been detected the oligonucleotide is degraded, for example with an exonuclease or by internucleoside cleavage. If the nucleotide incorporated for sequencing is linked to the synthesised polynucleotide by a degradation resistant bond, this nucleotide can not be removed, and thus the synthesised polynucleotide increases in length by one base. In another preferred embodiment the nucleotides to be incorporated into the synthesised polynucleotide is linked to the rest of the oligonucleotide by a degradation labile linkage such as a phosphoramidate linkage, which is labile to acid, or a ribonucleotide linkage, which is labile to alkali and to a range of RNAses. Therefore treatment with the degradation agent causes the rest of the oligonucleotide to be removed, and so the polynucleotide being synthesized increases in length by one base.

The design of the strategy is such that even though an oligonucleotide composed of several nucleotides is ligated, the sequencing proceeds on the basis of a single nucleotide and prevents the addition of more than one base at a time. This is achieved by defining only the base at the site of ligation, providing either a set of redundant, randomized bases or a set of universal bases or a mixture of the two in the rest of the oligonucleotide. The defined base interrogates the sequence of the template. The random portion stabilises the interaction with the template, provides an appropriate substrate for specific chemical or enzymatic reactions and provides sites for attachment of the label and terminator.

In each step of synthesis, four differentially labeled pools of oligonucleotides are used (preferably all pools are used simultaneously); with each pool being differently lableled from any of the other pools.) In each pool there is a defined nucleotide, A,C, G or T at the one terminus and the rest of the positions in the oligonucletide are randomized and/or are universal. The randomized/universal section ensures that sufficient length is available for stable annealing and ligation. The label can be attached at some point distal to the site of ligation. If it is used at the terminus then it can act as a blocker to prevent more than one oligonucelotide from being ligated.

This method can be adapted so that more than one nucleotide is incorporated into the synthesised polynucleotide by engineering the oligonucleotide so that the degradation labile intranucleoside bond is after two or more bases from the terminal which is ligated to the polymer. The method must ensure that there is base pairing between all of the bases which are incorporated into the primer, and the template, to ensure accurate sequencing.

It is not neccessary provide a fully degenerate set of probes based on the four natural nucleotides. Universal oligonucleotides, N-mers, "wild card" nucleotides, or "degeneracy reducing analogs" can be provided to significantly reduce, or even eliminate, the complexity of the probe mixture employed in the ligation step. It is recognized that universal bases do not bind to each of the four bases equally. However, as long as binding does occur, the strength of binding is not important. Mismatches at the sequences beyond the junction are irrelevant. It is important that the nucleotide at the junction is correct.

As with other embodiments of this invention the label may a fluorescent dye or a nanoparticle. However it may also be labelled with a mass tag and mass spectrometry could be used to read the identity of the added nucleotide. The approach can be applied with the bulk approach or at the single molecule level. It can be applied at the level of DNA colonies/clusters, in gels or surfaces, on microarray, microbeads, optical fibres, nanovials, microwells and on an ordered arrangement or on a random arrangement.

Phosphorothioate DNA and RNA, 2' -O-methyl RNA and methylphosphonate residues embedded in standard residues are capable of faithful information transfer. It has been shown that a range of modifications are compatible with information transfer. Phosphorothioate DNA is capable of supporting information transfer in non-standard backbone. (Thaler *et al*)

In one preferred embodiment the array is made in the 5' to 3' direction. The sequencing oligonucleotides is thiophosphorylated at the 5' end. After ligation, a 3'to 5' exonuclease is added. This degrades the nucleotides of the oligonucleotide until it reaches the phosphorothioate linkage which it cannot degrade. This leaves the incoprporated base with a 3' OH. This can then form another degradation resitatnt linkage with the next oligonucleotide thiophosphorylated at the 5'. end. This system is shown in figure 6.

A similar scheme can be carried out with the subtitution of the 5' thiophosphate with a methylphosphate, or a boranophosphate or any other modification at the 5' position that is compatible with ligation, and leads to the formation of a degradation resistant linkage.

Rapid thermocycling can be conducted which is useful for flushing off incorrect oligonucleotides from the ligation site, giving the correct oligonucleotrides to take their place.

### Ligation-Quencher sequencing:

A ligating oligonucleotide can also have a label and quencher. The quencher can be removed after a first step, by a mechanism that removes it only where a double stranded molecule has been formed. This can be by RNAseH cleavage or an RNA/DNAbase.

As described for polymerase extension, the provision of a quencher to the incoming ligation oligonucleotide will prevent non-specific binding and if desired will enable a closed system to be implemented in which the cleavage or ligation reactions are controlled by light or electrical pulses. Unlike the polymerase case, where the quencher automatically releases with the pyrophosphate after incorporation, the ligation scheme needs to be specifically engineered to implement a useful quencher system. One useful feature of a quencher system would be to ensure that the quencher is only removed when the oligonucleotide has annealed to the template polynucleotide. This quencher system can be implemented by either a cleavage based approach or a molecular beacon approach.

In one embodiment the invention the oligonucleotide comprises the structure:
Terminal nucleotide -N - nucleotide attached to a fluorescent label - M - nucleotide attached to a quencher
   wherein N and M are each independently a bond or at least one nucleotide; and M comprises a first degradation labile intranucleoside bond.
   In one aspect the present invention provides a method of sequencing a target polynucleotide comprising the steps of:
   (a) Carrying out template derived nucleotide synthesis by ligating an labelled oligonucleotide to a primer annealed to said target polynucleotide, wherein said ligation form a degradation resistant bond , and wherein said oligonucleotide comprises the structure:
Terminal nucleotide - N - nucleotide attached to a fluorescent label - M - nucleotide attached to a quencher
   wherein N and M are each independently a bond or at least one nucleotide; and M comprises a first degradation labile intranucleoside bond; and
   N comprises a second degradation labile intranucleoside bond, wherein said second degradation labile intranucleoside bond is resistant to the degradation agent used to degrade the first degradation labile intranucleoside bond;
   (b) Contacting said oligonucleotide with a first degradation agent;
   (c) Detecting the presence or absence of said labelled oligonucleotide;
   (d) Contacting said oligonucleotide with a second degradation agent;and
   (e) Repeating steps (a)-(d)

N and M can consist of a number of nucleotides, for example 2-15, or 4-10, or 6-8. However M should not be so large that the quencher does not act on the fluorescent tag. The important factor is that the oligonucleotide is not so large that the efficiency of the system is reduced. If M is one nucleotide then either of the bonds attaching it to the nucleotides either side can be the degradation labile intranucleoside bond.

In a preferred embodiment N also comprises a second degradation labile intranucleoside bond, wherein the second degradation labile intranucleoside bond is resistant to the degradationa gent used to degrade the first degradation labile intranucleoside bond. Again ifN is one nucleotide then either of the bonds attaching it to the nucleotides either side can be the degradation labile intranucleoside bond.

In the cleavage based approach, as shown in Figure 9, two independent cleavage systems are engineered into the incoming oligonucleotide. The quencher is attached to the oligonucelotide at the end distal to the point of ligation. Nucleotides or intemucleoside linkages are then provided that comprise the first cleavage system, This cleavage system must be one where cleavage only occurs once the appropriate kind of duplex has formed. For example 3' to 5' exonuclease activities of DNA polymerases and exonuclease III require a 3' recessed or blunt ended duplex in order to act. Also RNAseH only cleaves a ribonucleoitde linkage when it is base-paired with a DNA molecule (an RNA:DNA hybrid). The PN sysem could not for example be implemented here because acid will cleave the linkage and release the quencher regardless of whether the oligonucleotide has interacted with the template. Then after one or more nucleotides the label is attached to the oligonucelotide (biotin and NH2 residues can be incorporated into internal positions). Then the second cleavage system is at the point that after cleavage the register would be shift by the desired number of nucleotides. Hence, the first cleavage will be implemented to release the fquencher, allowing the label to fluoresce and be detected. Once detection has been completed, the second cleavage system can be implemented so that the label is removed and the sequence register is shifted appropriately. The second cleavage system muste be different to the first cleavage system but does not need to be constrained by the need to have a duplex as substrate So the PN cleavage system could be implemented here.

The second cleavage system may be any type of cleavage system disclosed in this invention apart from the type used for the first cleavage system. Other systems reistent to exonucelase activity such as PN, Methylphosphonate, LNA and other types of relevant bonds can substituted for the phosphorothioate linkage. The PN bond is compatible in this system because the degradation mechanism is exonuclease cleavage not acid treatment.

The quencher can be attached onto a normal nucleotide in an oligonulcoetide but there may be an ribonucleotide some where between the nucleotide attached to the quencher, and the nucleotide attached to the label. The label is closer to the ligation terminus, but it is separated from the incoporated nucleotide by degradation labile bond, i.e. a PN bond. Therefore a first treatment, such as RNAse, or alkali, will remove quencher. After detection, the label can then be reomved by a second treatmet to degrade the intranucleose bond..

In the opposite scheme, the PN bond will separate the quencher from the label. First alkali treatment will remove the PN bond then to remove quencher, then the label will be detected. The label and the rest of the nucleotide will be removed by alkali treatment which will cleave the RNA..

In another preferred method an exonuclease (exoIII for PN at ligation junction, T& exonuclease for RNA) can be used to degrade the hybridised DNA from the quenhcer to some internal point in oligonucleotide so that quencher can be removed but the label stays in place.

The internal point in the DNA can have degradation resistnat bonds such as PTO bonds, LNA, or methylphosphonate.

The Fluorophore-quencher can also be in a molecular beacon format. The beacon strand can be displaced by a stronger interaction leading to release of quencher from proximity of the fluorophore.

Thus in another embodiment the of the present invention the oligonucleotide comprises the structure:
Terminal nucleotide- N- nucleotide attached to a fluorescent label - L - nucleotide attached to a quencher
   Wherein N is a bond or at least one nucleotide; and
   L comprises a number of nucleotides which together form a hairpin structure, so the the fluorescent label is quenched when said oligonucleotide is not annealed to said template.

In a preferred embodiment N comprises a degradation labile intranucleoside bond.

N and L can be a number of nucleotides, for example 2-15, or 4-10, or 6-8.

### Chemical cleavage

The sequencing by ligation and exonuclease method described above involves two separate enzymatic steps. While it is certainly wise to include an enzymatic step for the sequence discrimination step of ligation, it would be better if the removal of n-mers could be achieved by a simple chemical treatment (e.g. the slide could just be dipped into a chemical bath for a few seconds.) The expense and time needed to perform an enzymatic reaction for degradation would then be eliminated.

In a preferred embodiment this can be achieved by using a PN bond to link the nucleotide to be incoporated to the rest of the oligonucleotide.

A 5' phosphate oligonucleotide containing a PN bond between terminal and penultimate nucleotide, is ligated with a 3' OH arrayed oligonucleotide. The PN bond is cleaved to leave a 3' phosphate on the arrayed oligonucleotide with a one nucleotide shift downstream in the register. However, an enzyme is required to remove a 3' phosphate before the next 5' phosphorylated oligonucleotide containing a PN bond can be ligated. This can be done by a Polynucleotide Kinase (PNK). The PNK can be mixed with the ligase so that only a single step is required. Alternatively this approach can be implemented using chemical ligation so does not require a PNK step nor expensive enzymes. It requires a 3' phosphorylated free primer. A 5' phosphorlyated incoming oligonucleotide with a PN bond between the first and second nucleotide is then ligated. Following detection the PN bond can be cleaved with acid and the register is shifted one nucleotide and a 3' phosphoryl terminus is regenerated.

A fully chemical sequencing method involves chemical ligation and chemical cleavage. Involving the steps:

### RNA cleavage sequencing

The method can also be achieved by using a ribonucleotide.

However, the RNA is cleaved at its 3' end. Therefore if the incoming oligo is the 3' end, and the ribonucleotide is at the penultimate position, cleavage will leave the terminal base, which can be a normal deoxy nucleotide, in position. This causes a one nucleotide shift in the register. However, a 5' OH will be generated which will need to be phosphorylated using Polynucleotide Kinase. This strategy will work with a regular orientation array bearing a 5' phosphate. The 5' phosphate can either be added during oligonujcelotide synthesis or post-synthetically by enzymatic phosphorylation using polynucleotide kinase. It should be noted that this method works in the reverse direction i.e. 3' to 5' compared to the PN method.

The degradation agent may be a chemical agent, for example, alkaline pH can mediate the cleavage reaction. The degradation agent may be an enzyme, For example there are two classes of RNAses that can be used; an RNAse H or a cocktail of RNA specific RNAses can be used. RNAse H degrades the RNA present in a DNA/RNA hybrid.

A single RNA position may be provided embedded in a deoxyribonucleotiode. Alternatively, while the terminal 3' nucleotide is a deoxyribonucleotide or a degradation resitant nucleotide such a s 2-0-methyl RNA, the rest of the oligonucleotide can be composed of degradation labile RNA nucleotides.

### 2 base sequencing

There are some embodiments of the invention that are compatible with sequencing two bases at a time. This can speed up the sequencing process. This can use 16 different labels to identify all of the possible nucleotide combinations in a pool.

One way of carrying out this two bases sequencing method is to utilise locked nucleic acids (LNA). It is known that LNA at terminal position only provides partial protection to exonuclease acitivity(DiGiusto and King 1994). However LNA at the penultimate position it provides complete protection. The reaction starts with a primer with a 3' OH. The incoming oligonucleotide has a 5' phosphate and an LNA nucleotide at the terminal position. An exonuclease is used to degrade the oligonucleotide, but it stops one nucleotide before the LNA residue. Hence after each ligation/degradation step two bases (the LNA and the next base) with an OH at the end will be added. Both the LNA and the second base form base pairs with the template.

Sequencing can be done on a two or higher number of bases per cycle, with any of the schemes discussed, simply by placing the degradation resistant or degradation labile nucleotide or linkage at the appropriate position. For example if a PN bond is placed at the 5^{th} position in the oligonucleotide then 5 bases will be added in the cycle. IT is important that all the bases that are added form the correct base pairing with the template to ensure the correct sequence is derived.

When single molecules are analysed at discrete foci on a surface, the four colours that are used to label single bases can also be combined to label all 16 possible combinations of 2 bases. The following list illustrates this:
AA red
CC green
TT blue
Gg yellow
AC red+green
AT red+blue
AG red+yellow
CA red green blue
CT green+blue
CG green+yellow
TA red green yellow
TC green blue yellow
TG blue+yellow
GA blue yellow red
GC red green yellow blue
GT red red red red
No incorporation, no colour

There are several types of other coding schemes that could be implemented as known in the art.

### Arrays

The methods of the present invention can be carried out on an array, as shown in figure 1. The term "array" as used herein relates to a spatially defined arrangement of one or more nucleotides in a pattern on a surface. The array can consist of individual nucleotides present at at least 96, 384, 536, 10,000, 48,000 or 192,000, 786, 000, 60 million discrete locations on a surface. The array is preferably formed on a chip. The array may be present within a microfluidic conduit. The arrays may also be on the bottom of microtitre plate or on flat bottomed microfuge tubes. These preferably have a bottom composed of high optical quality material.

The array can be a random array wherein the nucleotides are attached to the surface randomly. Alternatively the arrays can be spatially addressed. The nucleotides can be arranged in a grid pattern, with regular spacing between each nucleotide. The nucleotides can be located in a "spot" along with a plurality of other nucleotides of the same sequence. Alternatively the arrays can comprise DNA colonies.

Also the arrays can be composed of tandem copies of the same sequence within a single polymer as can be created by Rolling Circle Amplification (RCA) (Smirnov *et* a/).

The polynucleotides can be attached either directly or indirectly to the surface. For example an enzyme, such as a ligase or polymerase, ustilised in the process can be attached to a solid surface. The enzyme then binds the target polynucleotide, thus anchoring it to the solid surface.

Alternatively the polynucleotides can be captured by oligonucleotides which are attached to the surface. The capture can be by hybridisation of a single stranded oligonucleotide to a single stranded target or a single stranded region of a double stranded target. Alternatively, the polynucleotide or the surface immobilised capture probe, may comprise a sticky end or both may have a sticky end. The template and synthesized strand can be permanently linked to the surface by a ligation reaction. Alternatively the permanent fixing can be mediated by including a Psoralen moiety opposite a thymine residue and cross-linking with UV light.

Molecules can be attached to a solid surface by a number of methods that are well known to the person skilled in the art (such as those described by Fodor *et al* (199 1), Hegner *et al* (1993), or Finzi and Gelles (1995). Suitable methods of using nucleotides to form an array, and attaching nucleotides to an array are described in WO02/061126 and WO01/57248.

During array synthesis or the preparation of oligonucelotides to make the array UniCap Phosphoramidite (Glen Research) can be used for efficient capping in oligonucelotide synthesis. This will prevent undesired n-1mers (truncated oligomers) from participating in subsequent sequencing by synthesis reactions.

The surface is preferably glass, silica or a polymer such as PMDS or a Fluoropolymer. The substrate is preferably a glass slide, coverslip, silicon wafer, microfabricated chip or multi-well plate, such as a flat bottomed optical grade 96 well plate. The polynucleotides may be attached to material that coats the surface. For example aminosilane coated surfaces supplied by Corning Inc (USA) or Asper Biotech (Estonia) can be used. The surface may be coated with a gel material including agarose, polyacrylamids or a sol-gel. The polynucleotides may be attached to beads, particles, or structures such as nanobars or nanorods which may contribute to the generation or modulation of a FRET signal. The surface may be metalized with for example silver or gold particles to enhance a fluorescent or a raman signal (Malicka 2003; Kneipp 1999).

In addition, the surface or particles thereon may carry charge or be electrically biased or may be heated in order to control the sequencing process (Hamad-Schifferli, 2002). A charged surface is particularly useful to prevent non-specific interactions of nucleotides on the surface. Appropriate surface coatings include Polyethylamine as described by Braslavsky, 2003 and the DNA-bind slide available from VBC Genomics (Austria). An electric field generated at the surface is a useful way for controlling the attraction and repulsion of nucleotides at the surface (Asanov 1998; Sosnowski 1997)

Compared to the degree of parallelism currently available (96 Sanger sequencing reactions within individual capillaries on a state-of-the-art DNA sequencer) a whole wafer high-density oligonucleotide array has the capacity to analyse 60 million reactions (e.g. see www.perlegen.com website).

Until recently, the high cost of making individual photolithography masks meant that methods for making high-density oligonucleotide arrays were only available for mass production of arrays and were not accessible for the individual design of single arrays. However, the application of a Texas Instruments' digital micromirror device (DMD) to array synthesis has made it much more straightforward and cheaper to specify individual arrays. The DMD is a chip comprising an array of 786,000 micromechanical aluminum mirrors, where each mirror is individually addressable. Using these tiny aluminum mirrors to shine light in specific patterns, coupled with the photo deposition chemistry, arrays of oligonucleotide probes are produced. Several companies and laboratories have implemented this technology, notably Xeotron and Nimblegen. A fully integrated benchtop device for making, hybridising to and analysing high-density arrays can streamline an entire miroarray experiment to within one day, (e.g Geniom one; Baum *et al*)*.* The Geniom one uses the DMD to create an array by the spatially-selective deprotection of photolabile protecting groups on DNA chains growing on a surface. Each new array design can simply and rapidly be specified by software and there is no need to make photolithography masks. Arrays can be made such that the sequencing can be initiated either with an array of oligonucleotides directed to specific regions in the genome or with an array of n-mers (Gunderson *et al,* 1998) which will initiate the process at any position which seeds hybridisation. Presently, Geniom one is configured to synthesize 48,000 oligonucleotides. However, it is possible to synthesize at least 192,000 sequences on one chip in one synthesis run. All the synthesis, hybridisation and washing steps can be undertaken within the microfluidic channels of the chip provided by the manufacturer. The benefit of this system is that it can rapidly iterate array synthesis based on information that is obtained.

In one embodiment the method is carried out using an array wherein multiple copies of one primer are located within a localised area. The combined signal from all the nucleotides incorporated is detected i.e. the "bulk" signal is detected. The signal detected will be that relating to the nucleotide that is incorporated the most. In the embodiment wherein the nucleotides are all labelled identically and added individually, the strongest signal will be obtained when the nucleotide which is the correct base pair corresponding to the next base in the template is used. In the embodiment wherein the nucleotides are differentially labelled, the fluorescent label that is detected corresponds to the fluorophore used to label the next nucleotide in the sequence. The signal from any wrongly incorporated nucleotides, or errors will be diluted by the strong signal from the correctly incorporated nucleotides. When a sample is heterozygous at a particular locus two colours will be equally represented.

A plurality of individual molecules or units can be analysed with microarray spots, DNA colonies or Clusters, gel or bead immoblised colonies, or RCA foci.

The arrays may be created on surfaces which are compatible with enzymatic reactions and have low absorption of fluorescent reaction components. The surface can be coated with agaorse, polyacrylamide, sol-gel, polyelectrolyte multi-layers, Bovine serume albumin/biotin/streptavidin coating or various types of polymer matrix.

### Single molecule detection in Sequencing

Single DNA molecule imaging can be used to detect the template and/or incorporations as a point-source of fluorescence, for each individual molecule.

Molecules within arrays are distributed at a density at which substantially all molecules are separated by a distance greater than that required for resolving them as separate entities (defined by the diffraction limit of light). Then, instead of analysing a single intensity value due to the combined signal from thousands of molecules, a "digital" signal from each molecule can be individually assessed. This enables heterogeneous reactions within a microarray spot, which would ordinarily be masked by the signal averaging of ensemble methods, to be detected.

Determining which nucleotide has been incorporated in a single molecule allows multiple copies of a polynucleotide to be sequenced individually, in parallel. The present invention enables, for example around 10³ sequence passes (or sequencing redundancy) within a microaray spot. If a thousand copies of a polynucleotide can be sequenced at the same time, it is effectively equivalent to repeating the sequencing a thousand times. This considerably reduces the amount of time required to carry out this work, as compared to the traditional Sanger dideoxy techniques. It also provides increased confidence levels. This method eliminates the need for costly amplification steps, and can be used to provide haplotype information.

The single molecule sequencing approach developed previously involves a "random" display of molecules to be sequenced without any deliberate organization of the molecules by spatially addressable arrays. The methods of the present invention can be applied to such types of random arrays of single molecules. In such a set-up although there may be several other copies of the same sequence present elsewhere, at undefined positions on the surface it will be difficult to extract statistical confidence in a sequence due to heterozygocity and the presence of other closely related sequences. Therefore, each molecule is essentially sequenced with one pass only. By contrast single molecule sequencing within spatially addressable microarrays enables for example, around 10³ molecules of the same species to be sequenced within each array spot. Hence, the confidence levels with which the sequence will be obtained will be unprecedented. If related but different sequences are captured within a spot, their identity will become apparent after several cycles of base addition. If the sample is heterozygous, then the presence of two species within the spot will be seen.

Although the methods of the present invention are preferably carried out on a solid surface, they can also be conducted on molecules which are free in solution for example in the wells of a microtitre plate or within micro or nano-scale vials, wells or structures (Levene *et al* 2003). The method can also be carried out in nanochannels (Cao *et al* 2002; Tegenfeldt *et al* 2004)

The methods of this invention are preferably undertaken on surfaces because it is easier to organise and monitor reactions on a surface than reactions freely diffusing in solution. However, when the molecules are immobilised on beads which are able to diffuse, one can take advantage of the improved reaction kinetics of solution phase reactions. A sequence strategy has been described for molecules immobilised on beads (Brenner *et al* 1999) and the methods of the present invention could be applied on this platform. Where the beads are magnetic, the additional functionality can be used to facilitate the process. In addition, the molecules could be trapped in solution space via optical. Magnetic or electrostatic traps.

As the invention can be applied in a single molecule detection mode it is very sensitive and can be performed on small amounts of sample material. Hence the invention can also be applied in a context where one or very few molecules are available, such as from ancient DNA or a forensic sample.

Although the invention can be carried out on a purified fragment of a polynucleotide, it offers particular advantages for sequencing polynucleotides directly from a complex mixture such as sheared/fragmented genomic DNA, a mRNA population or a population of fused mRNA-polypeptides.

Also this method does not necessarily require PCR to enrich or amplify the sample DNA. In particular, locus-specific PCR reactions can be avoided. In some instances it may be desirable to perform whole genome amplification before sequencing. To avoid errors due the complexity of genomic DNA it may be useful to sequence the genome in different fractions. When the goal is resequencing, the possibility of complications due to duplicated regions of the genome caneb taken into account. This is needed particularly when the invention is implemented in bulk mode. When the invention is implemented in single molecule mode the identity of each individual molecule is fully determined regardless and hence a contaminating sequence can be identified. However to avoid unnecessary sequencing repetitive DNA can be suppressed by for example subtraction with Cot-1 DNA. The invention can be implemented on spatially addressable arrays so that different regions of the genome or different species in a mRNA population are captured at specific known locations. One advantage of this is that capture probes provide a certain length of sequence information even before the sequencing by synthesis data is obtained.

Methods of this invention can be carried out in in a mode where reaction components for the different steps of the reaction are provided at separate stages. The methods can also be carried out in a "homogeneous" way or mode, where all the components required for the reaction are provided in the reaction vessel from the start. Then cyclical electromagnetic modulation, for example for cleaving a linkage provides a clocking mechanism for shifting the sequence register. Furthermore, some of the methods of this invention can be carried out in real-time, by providing reaction components and then continuously monitoring the reaction. Preferably for this embodiment the signal is detected by a FRET mechanism as described below.

### Sequencing by FRET

In fluorescence resonance energy transfer (FRET), a donor fluorophore molecule absorbs excitation energy and delivers this via dipole-dipole interaction to a nearby acceptor fluorophore molecule (Stryer, L. and Haugland, R.P. 1967.). Fluorescence resonance energy transfer can be used to cut out background fluorescence in single molecule experiments (Braslavsky *et al* 2003). Recently, a new way of using FRET in a DNA assay, termed iFRET has been introduced in which the donor dye is an DNA intercalating dye that is used to stain DNA (Howell WM *et al.* 2002, Japanese paper). iFRET is reported to give fluorescence values that are 2.5 times greater than those obtained from the intercalating dye alone, and more than 40 times greater than those from conventional FRET. It is suggested that the reason for the difference may be that the iFRET system involves the channelling of an accumulation of energy from a chain of donor dye molecules (in contrast to a single donor in the FRET system) into the acceptor moiety, which is then able to re-emit energy unhindered. Double-strand, DNA-specific intercalating dye (e.g., SYBR Green I) has been used as a FRET donor, with a conventional FRET acceptor.

A FRET mechanism can be implemented with the sequencing by synthesis methods described in this invention. One embodiment of the present invention involves the detection at the single molecule level, using FRET between two or more FRET partners. The FRET partnership system comprises two or more partners each attached to a reaction component selected from the group comprising nucleotide, the template, the polymerasing agent or any other reagent involved in the polymerization reaction. Donor-acceptor fluorophore pairs are chosen so that the emission spectrum of the donor overlaps with the excitation spectrum of the acceptor; many different combinations of available fluorescent labels can be used.

In one preferred embodiment the FRET means of detection is utilised in a method wherein the labelled nucleotide is detected as it approaches the target DNA molecule. As the labelled nucleotide is brought into the proximity of the target polynucleotide during polymerisation, the FRET reaction occurs between the label on the nucleotide and a FRET partner. This reaction can be detected. The FRET label is attached to the nucleotide through the beta or gamma phosphate groups. These phosphate groups are removed as the nucleotide is added during extension, so effectively the detection of the label, the extension, and the replacement of the labelled nucleotide with an unlabelled nucleotide occur almost simultaneously. When the nucleotide has been incorporated it is no longer labelled. The released pyrophosphate is free to diffuse out of FRET range.

Preferably the repertoire of nucleotides, e.g. adenine, cytosine, guanine and thymine are each labelled in a way that their FRET signals can be distinguished from one another.

In another preferred embodiment FRET occurs between a DNA stain (e.g. an intercalating dye) bound to DNA and one or more FRET partners attached to another polymerisation reaction component such as the nucleotide or polymerising reagent. The bound DNA stain may act as FRET donor or acceptor. It is simple to add a DNA stain that incorporates at multiple positions along a template molecule so that it can contribute to a FRET reaction anywhere along the extending chain. One of the FRET partners may be the fluorescently labelled nucleotide, which is utilised to extend the polynucleotide being synthesised. The fluorescent label may be directly or indirectly attached to nucleotide, and it may be a nanoparticle. Preferably the DNA stain is not the first FRET Donor as this could lead to it's wholesale photobleaching; although this can be minimised with judicious choice of antifade composition. Several DNA stains are available for staining double-stranded DNA and a few of these are also able to stain single-stranded DNAs relatively efficiently, e.g. SYBR Gold. However, many dyes can cause light-mediated strand breakage tooccur. The dye Sytox green is relatively resistant to this.

Alternatively, or additionally the FRET partner can be attached to the polymerase, for example the DNA polymerase. The FRET label may be in the form of a semiconductor nanocrystal/Quantum Dot, as these do not photobleach which is important as it is desirable to retain the same polymerase throughout synthesis.

Multiple FRET interactions can take place when the excitation and emission spectrum for FRET partners overlap. The first FRET partner is excited at one wavelength, and its emission wavelength overlaps with the excitation wavelength for the second FRET partner. The second FRET partner has an emission wavelength which overlaps with the excitation wavelength for a third FRET partner. In this way a chain of energy transfers can take place, when the FRET partners are within FRET range and the first donor has been excited. This can result in a large stokes shift i.e. large separation of excitation from emmision. This allows the signal to be read at a wavelength far removed from to the original excitation wavelength, which is advantageous for eliminating bleed-through from the excitation source into the detection channel. Importantly, this method also ensures that all of the components (the target polynucleotide, the labelled nucleotide and the polymerase) are all in close proximity. In some instances an anti-stokes shift may be utilised.

Howell *et al* describe a system in which the intercalating dye acts as donor. A Single molecule system may involve syber green 1 as the donor and a rox labelled nucleotide as the acceptor. As Quantum Dots can be excited at various wavelengths, when they are used as the acceptor, donors emitting at various wavelengths can be used, e.g. DAPI or SYBR gold. Alternatively, the incorporated fluorescent nucleotide or a fluorescent nanoparticle can act as the donor and an intercalating dye such as PO-PO3 can be used as the acceptor (Nakayama *et al* 2003). The Quantum Dot can be excited at a wavelength far removed from the acceptor dye. The signal produced would be due to the localised excitation by the Quantum Dot of a few fluorescent dyes in its locality. Following detection of the FRET signals, an image of the polynucleotide polymer can be taken by exciting the DNA stain directly. Alternatively a stretched out target polymer can be viewed by DIC (Differential Image contrast). The FRET signal can then be superimposed on the polynucleotide polymer image, to determine where incorporation has occurred.

Because energy transfer to the acceptor is from a highly localised source, background fluorescence from anything beyond the FRET range, which is about 10nm, does not contribute to background fluorescence. Hence, FRET would enable reactions to be monitored continuously without the need for washing away of unbound fluorescent dyes or nanoparticles. This would enable addition of more than one nucleotide to be detected in real-time. The system can be homogeneous in that all that is needed for the reaction can be placed in the reaction vessel at the start of synthesis. It would be desirable to retain some form of agitation or mixing of the reaction solution to enable pyrophosphate to diffuse out of FRET range after it has been released.

In accordance with the above in one aspect the invention provides a method of sequencing a target polynucleotide, comprising the steps of:
(a) carrying out template derived polynucleotide synthesis utilising a nucleotide labelled with a FRET partner and at least one other polymerisation reaction component labelled with a FRET partner_{;}
(b) determining the nucleotide incorporated by detecting FRET interactions; and
(c) repeating steps (a) and (b).

Preferably this method is used to carry out real-time monitoring of the sequence.

The polymerisation reaction components include the polymerizing reagent and the template polynucleotide. Preferably a DNA stain is used to label the template and a Quantum Dot is used to label the polymerizing agent.

A nucleotide that may be temporarily resident within the FRET range of a polymerasing agent or a template molecule, may or may not get incorporated depending on whether it is the correctly matched nucleotide for the position in question. This temporary resident of the FRET locality must be distinguished from a nucleotide that is actually incorporated. This can be done by utilising information gathered prior to the reaction about, for example, the longevity or strength of the FRET signal depending on whether it originates from a nucleotide temporarily resident within the FRET locality or a properly incorporated nucleotide. WO00/36151 describes a mechanism in which the dye attached to the nucleotide remains quenched by a quencher until incorporation of the nucleotide occurs at which point the quencher becomes detached and allows the dye to fluoresce freely. The drawback of this approach is that there is likely to be loss of quenching which is not due to loss of quencher but is due to thermal or structural fluctuations or photobleaching. An alternative way of measuring incorporation in the context of the present invention is by detecting quenching/de-quenching or preferably a wavelength shift with a FRET partner which occupies a different reaction component than the nucleotide itself. For example, the emission due to the FRET partner on the template may be modified by a FRET Partner on the beta or gamma phosphate of the nucleotide. When the nucleotide is incorporated and pyrophosphate is released, the FRET interaction is abolished and hence a fluorescent property of the FRET partner on the template is modified, e.g., it emits fluorescence at a shifted wavelength. The first donor in this scheme may be a Quantum Dot attached to the polymerizing agent and the whole process may be designed to have multiple FRET interactions which are able to be monitored in real time, by using for example an image splitter such as the Quad-view from Optical Insights Inc. (USA).

Appropriate anti-fades can be used to attenuate photobleaching. This can include the provision of Oxygen scavengers and reducing agents such as DTT and 2-Mercaptoethanol.

TopoTaq (Fidelity systems) is resistant to common inhibitors of DNA polymerases, such as DNA stains such as SYBR green 1 and II and SYBR gold.

### Linear Polymer Display

Genomic sequence would have much greater utility if haplotype information (the association of alleles along a single DNA molecule derived from a single parental chromosome) could be obtained over a long range. This is possible by combining the SbS process of the present invention with the single molecule display of elongated linear genomic DNA described in WO02/074988. Here each template molecule is sorted on the array, and combed to provide a linear display of sequence along its length. Polymerisation can then be seeded at multiple positions on each linear molecule, e.g. optimised to be every 10kb or 50kb apart. The incorporations are monitored as slowly migrating point sources of fluorescence along the linearised DNA polymer. The introduction of nicks in the double stranded DNA is sufficient to prime synthesis with certain DNA polymerases. This involves strand displacement and enzymes such as phi29 DNA polymerase are particularly well suited to this. Enzymes such as DNAs1 can used to introduce nicks. A particularly useful nicling enzyme is the restriction endonuculease, Nb.Bpu10I which that cleaves only one strand of the DNA within its recognition sequence on double-stranded DNA substrate.

Various DNA stains can be used to visualize the DNA polymer. Various dyes are sitable for this including YOYO-1, POPO-3, SYBR Green and SYBR Gold which is particularly useful. Low concentrations can be used which are more suitable to enzymatic reactions. Single stranded DNA can also be labelled. Sytox Green is another useful dye as it resistant to light induced breakage of the DNA polymer. A dye can therefore be used to confirm that it is the template polynucleotide that is being sequenced, as opposed to some other contaminant. Rolling circle amplification can be performed on a circularized target. The resultant tandem copies can be combing and sequencing by synthesis can be viewed at each position, as shown in figure 2. In addition to sequencing by synthesis methods of this invention, sequencing can also be conducted by adapting other available methods, such as those described in WO97/46704.

### Resolving ambiguities in the sequence

If the sequencing approaches are carried out in a microarray format and the array making and sequencing is iterated, then ambiguities in the number of bases at any particular position can be resolved by making probes that would address each of the suspected sequence possibilities in the next array synthesis.

The results can be displayed with confidence levels for each base and where bases have been sequenced with low confidence, they are labelled with a confidence rating. This confidence rating can be taken into account when the sequence information is used, for example in genetic studies. Furthermore, when the task is re-sequencing, for example in humans, rather than *de novo* sequencing, the consensus sequence and the prevailing information about common SNPs and their frequencies in different ethnic groups will aid in deciding the correct sequence in a particular individual's sample. an RNA/DNA base.

### Sequencing to obtain gene expression information

The method of the present invention can be adapted to obtain gene expression data, particularly from a single cell. Once a certain length of sequence information has been obtained, it can be used to identify the mRNA species. The method can be modified for sequencing mRNA. Thus in one embodiment the target polynucleotide comprises mRNA. The mRNA can be hybridised to primers which are designed to hybridise to any mRNA molecule. For example, primers can be designed to hybridise to all sample mRNA species at a specific point in the mRNA primary structure. This point could be the polyadenylation signal, AAUAAA, the Poly A tail at the 3' end or at the 5' end or the cap structure at the 5' end or a specific sequence clamped onto the 5' or 3' end. Preferably the primers are attached to a solid surface, and more preferably form an array.

Thus in one aspect the present invention provides a method of sequencing mRNA comprising:
a) contacting an array of probes designed to hybridise to mRNA molecules with a sample of mRNA under conditions whereby the mRNA will hybridise to said probes; and
b) sequencing said mRNA utilising a method as descibed herein

### Bisulphite sequencing

The methods of this invention may be preceded by bisulphite conversion to determine methylated status of a sample.

### Co-sequencing two samples to find the differences between them

The DNA or mRNA from two or more individuals or populations can be compared by differentially labelling each template (i.e. labelling the template with a different label for each population or individual), immobilising them on a surface and then sequencing them simultaneously. The templates can be labelled, for example by attaching an oligonucleotide containing different fluorescent dyes by using RNA ligase.

The templates can be immobilised by attaching the labelled nucleotides to a surface as described above. The templates can be used to form an array. Alternatively the templates can be captured on to an array. This can be done for example, if the templates nucleotides are allowed to hybridise to primers which themselves form an array.

After, the templates are immobilised on the surface, the foci for each sample will be detected and recorded. Following this, the label can be photobleached and the sequencing can commence.

### Primer Considerations

In a double stranded molecule or when a primer is annealed to a single stranded molecule, there are two 5' ends and two 3' ends. Measures need to be taken so that there is only the possibility of chain growth at a single 3' end or a single 5' end depending on mode of synthesis. Otherwise extension from a non-desired termini may complicate the analysis of the desired termini. When the target is immobilised on a surface by one of the ends, this end no longer participates in extension. For example a polynucleotide template can be immoblised to a surface via its 3' end and so then the only 3' end available for extension is the 3' end of the primer. In other instances the template may be captured by an immoblised primer in which case the 3' end on the template polynucleotide needs to be inactivated. This can be done by for example by ligating a blocked oligonucleotide to the end or extending with a Terminyl transferase, using ddNTPs prior to annealing to the primer. In fact Terminyl Transferase can be used to tail a template polynucleotide with a homopolymer sequence to facilitate annealing to an appropriately designed primer.

The possibility of forming primer-dimers and other structures leading to artefacts must be avoided. This can be done by correct design of primer/priming site, where this is possible. Also primer modifications that can specifically minimise such artefacts, such as Fimers™ (Fidelity systems, USA) or Super G™ (Epoch, USA) can be used. Also already annealed primers, such as those generated by a nick are preferably in this respect. The formation of artefactual extension in the absence of enzymes, that cannot be denatured by extreme denaturing conditions, suggests that covalent (or covalent-like) interactions can occur by non-enzymatic means in certain situations. Such errors must be eliminated from the analysis.

There are various types of primer constructs that can be used:

### Capture by single stranded oligonucleotides

If a repertoire of single stranded oligonucleotide probes are arrayed or spread out onto a surface they can serve as capture probes either to target molecules bearing sticky ends (to which they may become ligated) or by sequence-specifically binding along a target single or double-stranded molecule under appropriate conditions.

Quite often enzyme preparation have functionalities in addition to the one that is desired. For example, an enzyme may have an exonuclease as well as polymerase activity. Or the enzyme preparation may have a contaminating activity present. Measures may need to be taken to prevent these from having affect.

The ends required for extension may be present at positions additional to the ones that performing the extensions reactions that are desired. For example, in addition to the primer having a free 3' or 5' end, the target molecule may will have 3' and 5' ends. So for example in the case of polymerase extension of 3' ends the extension could be seeded by the 3' end of the template (this could occur by self-priming or by the non-intended template site). Therefore measures can be taken to block sites that could lead to non-intended extension products. This may entail for example ligation of a blocking moiety to the 3' end of the templates, for example by T4 RNA ligase, or terminal transferases utilising ddNTPs. The 5' end can be blocked by ligating a universal blocking sticky end.

One mechnsims to block an end is if it engages in a ligation reactions to primer complex, as in the case, described. Also in the stem loop case described, one of the ends of the template itself becomes the legitimate primer.

The steps of processing the template to prevent extension in the 3' and/or the 5' end, and use in any of the schemes described in this invention.

The nucleotides that are available are not 100% pure. Often the other bases contaminate. Therefore where a labelled reaction gives a particular signal which would be expected to be due to a particular base, in a minority of cases this might in fact be due to a different base. This different base can incorporate at a low rate especially if nucleotides are in limiting concentrations. To avoid this, dummy templates can be provided in solution, with which these nucleotides preferably react This is particularly important when all 4 bases are not added simultaneously and the sequencing scheme involves addition of 3 or fewer of the 4 nucleotides.

This is a particular problem when a random array is used. However, when a population of molecules is examined within a spatially addressable array then such infrequent errors can be discriminated as such.

It is suggested that random arrays are not amenable to de novo sequencing and should be restricted to re-sequecing. The methods where a plurality of copies of a molecule are found in an identifiable group, de novo sequencing can also be done.

### Capture by sticky probes

An array of "sticky" probes can be created by designing and purchasing customized oligonucleotides (e.g. from IBA-GO.com). Firstly, a binary oligonucleotide repertoire, A is created which partially contains a fixed sequence and partly contains a randomized sequence. A second oligonucleotide is provided, B which binds by complementary base pairing to only the fixed sequence on oligonucleotides of the repertoire, A. This process may be carried out entirely in solution and then the complex spread out on the surface. Alternatively, one of A or B is first spread out on the surface and then the other is reacted with it. Both the above procedures are done under conditions that enable annealing/hybridisation, for example in 4XSSC 0.2%Sarkosyl or 3.5M TMA at a temperature determined by Tm. The binding of oligonucleotide pool A with oligonucleotide B creates a repertoire of cohesive or sticky ends. These sticky ends are able to bind the termini of DNA molecules. Gunderson *et al* describe how this can be done on a spatially addressable array.

The sticky probes ensure that as the new strand is synthesized both it and the template remain in close proximity irrespective of whether harsh treatments that may denature hydrogen bonds, are performed. If this was not the case certain harsh treatments may delocalise one strand from the other and undermine the continuity of sequence acquisition.

The template can be joined at one end with a sticky end at the other. The sticky end interacts with the capture probe but only one of the ends of the sticky probe forms covalent interaction. The other end may fall short of the ligation junction or may not have an appropriate end for a ligation for example it may possess a 3' phosphate or lack a 5' phosphate. However it could be made to act as a nick to initiate strand displacement synthesis. The joining of the template can be easily done when the template is a cDNA as this occurs naturally (see Maniatis) when mRNA is being copied with a reverse transcriptase.

### Permanent capture mediated by stem loop structure

Target molecules can be captured to sticky ends in a similar way to the above but the non captured end can be joined together via a hairpin, a loop or a linker/spacer. This also overcomes the problem of having two active ends for extension. The linking moietie(s) can also serve as immobilizers. For example, phosphorothioate moieties in the loop can react with an aminated surface. This can also be achieved by the inclusion of, for example, a NH₂ group between two spacers or the inclusion of NH₂-dT.

### Permanent capture mediated by Dendrimer

Capture can be via an artificial hairpin composed of a 3'-5' oligonucleotide connected to 5'-3' oligonucleotide, as illustrated in figure 10. This can be synthesized by using an asymmetric dendrimer phosphoramidite which is available from Glen Research. One arm is protected by DMTO and the other arm is protected by Fmoc. Hence it is possible to synthesize an artificial hairpin, by synthesizing an oligonucletide sequence on one arm in one orientation and a complementary oligonucleotide in the opposite anti-parallel orientation so that a hybrid can form. This is an ideal substrate as it can be linked to the surface and the annealed arms can be used to capture the target (for example, a sticky end may be generated byone arm overhanding the other). Alternatively this construct can be ligated or annealed to a template molecule in solution before immobilization.

### Permanent capture to oligonucleotides connected by streptavidin

The template and primer strands can be connected by interaction of biotin molecules on the appropriate ends (for example the 5' end of a primer and the 3' end of the template) in a manner that they form interactions with the same single streptavidin molecule.

### Permanent capture by mediated by Psoralen

Alternatively the permanent fixing can be mediated by including a Psoralen moiety opposite a thymine residue and cross-linking with UV light. Sticky probes created by annealing of oligucleotide can be permanently connected in this way, hence ligation need only be with one of the strands of the stick end. In addition when a single standed polynucleotide is captured by single stranded capture probe can become permanently linked when one of the strands is modified with a Psoralen and the other place a thymidine nucleotide in its close proximity in its proximity. Other crosslinking systems can also be used.

### The problem of secondary structure

Single stranded polynucleotides can form intramolecular structure which can obstruct the binding of a primer or the progression of a polymerase. Also sometimes the template can fold in such a way that non-contiguous sequences are juxtoposed, which can lead to error in sequencing. To avoid this, extension can be carried out with a thermostable enzyme at a relatively high temperatures at which the intramolecular interactions are shortlived and unstable. For example, ligation can be done at 65 °C and polymerase extension can be done at 58 °C. Thermocycling can also be done which is particulary useful for a ligation based approach. In addition certain polymerases are compatible with denaturants such as Urea and DMSO. In addition single strand binding proteins such as E.Coli single-strand binding protein (SSB) and T4 gene 32 protein can be added; these have been shown to facilitate polymerase action. One other means is to perform a prior copying reaction, such as strand displacement amplification or PCR in which one or two of the native nucleotides are replaced with nucleotide analogues that cannot pair with each other, but are able to act as a template for extension with a different set of nucleotides. If denaturants are added then the primer and template must be held by bonds that can withstand the denaturation steps. For example, the primer may be composed of LNA which can form highly stable interactions. Alternatively they can be held together by bonds in addition to Watson-Crick bonds. For example, a covalent linkage or a streptavidin-biotin interaction, However, the problem of secondary structure can be prevented from occurring if the target is substantially or completely double stranded. This is the case if the primer extension is initiated from a nick. Strand displacement synthesis can be conducted by methods known in the art (e.g. Paez *et al* 2004).

### Detection schemes and instrumentation

The images of the polynucleotides are projected onto the array of a Charge-couple device (CCD) camera, from which they are digitized and stored in memory. The images stored in memory are then subjected to image analysis algorithms. These algorithms can distinguish signal from background, monitor changes in signal characteristics, and perform other signal processing functions. The memory and signal processing may be performed off-line on a computer, or in specialized digital signal processing (DSP) circuits controlled by a microprocessor.

When the base-by-base incorporation of labelled nucleotides is monitored on molecules in bulk (i.e. the combined signal from a population of polynucleotides within one spot on an array is measured), the established methods for scanning microarrays can be used to monitor incorporation at microarray spots after each base addition. For example, a Genepix scanner (Axon instruments) or a Scanarray (Packard) which can be linked to four different laser lines can be used.

When individual molecules are analysed directly, then wide field CCD imaging is used. CCD imaging enables a population of single molecules distributed 2-dimensionally on a surface to be viewed simultaneously. Although microarray imagers based on epifluorescence illumination and wide field imaging are available, the optics and range of stage movement of these instruments does not enable single molecules to be monitored across large areas of the slide surface. Typically, wide-field illumination schemes may involve illumination with a lamp, a defocused laser beam or by an evanescent field generated by Total Internal Reflection of a laser beam. The field that can be viewed is determined by the magnification of the objective, any magnification due to the C-mount and, the size and number of pixels of the CCD chip. A typically microarray spot can be viewed by either a 40X or 60X objective depending on CCD camera and C-mount. Therefore to view large regions of a slide (several cm²) multiple images must be taken. A low noise high sensitivity camera is used to capture images. There are several camera models that can be used; Cooled Micromax camera (Roper scientific) controlled by MetaMorph (also MetaView software; both from Universal Imaging). MetaMorph can be run on a Dell OptiPlex GX260 personal Computer.

### Microarray spot-finding and single molecule imaging within microarray spots (Figure 11)

MetaMorph's optional microarray module and a low magnification objective can be used to locate spots before taking a CCD image of each of the spots using higher magnification. As the signal from the spots containing singly resolvable molecules is very low under low magnification, a marker dye, which emits at a different wavelength to the sample emission can be included in the spots to help locate them. The objectives need to be of high numerical aperture (NA) in order to obtain good resolution and contrast. The integration of an autofocusing capability within the procedure to maintain focus as the slide is scanned, is useful especially when Total Internal Reflection Fluorescence microscopy (TIRF) is employed. Software can be used to control z movement (integral to motorized microscopes) for the purpose of autofocusing. Images of microarray spots can be obtained by x-y movements of the sample stage (e.g. using Prior Scientific's Proscan stage under MetaMorph control). To avoid photobleaching it is advisable to use a shutter (e.g. from Prior Scientific) to block off illumination while moving from one spot to another. A controller can be used to control x-y stage, the filter wheels and shutter, (e.g. Prior Scientific ProScan).Once the spots are found, their coordinates are recorded by the software controlling the instrument and then after each base addition, a CCD image is taken of each spot of the microarray. In addition to the instrument being used for looking at a microarray where template molecules have been captured by probes, a large number of samples can be gridded and then the instrument can be used to analyse each spot. The samples may be individual nucleotide populations or a set of differentially labeled nucleotide populations.

Alternatively, a commercial single molecule reader especially designed for genomics, the CytoScout (Upper Austrian Research, GmbH) can be used (Hess *et al* 2004). This system can identify areas or interest by doing a fast scan (at high magnification) before performing a slower acquisition of with single molecule sensitivity of regions of interest, e.g. DNA clonies/clusters or microarray spots. The CytoScout can provide 50-fold improvement of signal to noise compared to a conventional scanner.

### Epifluorescence Microscopy

Images of single molecules labeled with a single dye molecule can be obtained using a standard epi-fluorescence microscopy set up, using high numerical aperture (NA) objectives and a high grade CCD camera. However, the image can be hazy. In order to obtain a clearer image it is preferable to use deconvolution software to remove the haze. Deconvolution modules are available as drop-ins for MetaMorph software. When the single molecules are labeled with nanoparticles the camera and objectives may be of a lower grade and oil-immersion objectives may not be required.

### Total Internal Reflection Fluorescence Microscopy (TIRF)

TIRF enables very clean images to be obtained, for example using off the shelf system four Objective style TIRF (such as those produced by Olympus or Nikon). A full description can be found in the brochure at the following website: www.nikon-instruments.com/uk/pdf/brochure-tirf.pdf. Objective style TIRF can be used when the sample is on a coverslip. However, it is not compatible when the sample is on a microscope slide. For this either rPrism type TIRF (AJ Lacey) or a condensor based TIRF using a high NA condensor (Olympus, Japan) must be used to create TIRF. Although the above describes use of the system on an inverted microscope, an upright microscope can also be configured in an appropriate way, for example as described by Braslavsky *et al* (2003).

### Multi-colour single molecule imaging

When the sequencing strategy involves the sequential addition of each of the four nucleotides all labeled with a single fluorophore such as Cy3, then a single CCD image is taken after each base addition. However, if each nucleotide is differentially labeled (i.e. each nucleotide type is labelled with a different fluorophore) and added simultaneously, then the signal from each of the differerent fluorophores needs to be acquired distinguishably. This can be done by taking four separate images by switching excitation/emission filters. Alternatively, an image (Wavelength) splitter such as the Dual View (Optical Insights, Santa Fe, NM) or W View (Hamamatsu, Japan) which direct the light through two separate bandpass filters with little loss of light between them, can be used for imaging two different wavelenghts onto different portions of a CCD chip. Alternatively the light can be split into four wavelengths and sent to the four quadrants of a CCD chip (e.g Quad view from Optical Insights). This obviates the need to switch filters using a filter wheel. A MetaMorph drop-in for single image dual or multi-emission optical splitters can also be employed. Image splitting can be used to monitor FRET.

Four fluorescent labels need to be used that can be properly distinguished from each other. Ideally crosstalk between one dye and another should be kept below about 30-40% in order to be able to adequately separate them via thresholding and software manipulations. One combination that is used is Fluorescein, Cy3, Texas Red and Cy5. However, these dyes are difficult to resolve, although a commercial system, the Genorama, from Asper Biotech (Estonia) is able to resolve them using very 4 seprate lasers and very narrow bandpass filters. Other combinations that are easier to separate include, Fluorescein, Cy3, Cy5 and Cy7, or Coumarin, Fluorescein, Cy3 and Cy5, or dyes with similar wavelengths to the each of these. As there are several varieties of Quantum Dots available commercially with wavelengths ranging from 525nm to 800nm (Quantum Dot Corp, Palo Alto, USA), there are several combinations that can be differentiated. Chroma ScienTechnology are able to custom design filter combinations that can resolve these four colours. There are already commercial combinations available that can separate 4 wavelenghts, e.g. the 8400 series Quad Filter Set with single band excitation filters for DAPI/FITC/TRITC/Cy5™ (Chroma Technology, USA).

### Monitoring sequencing on single molecules randomly distributed on a surface

As an alternative to microarray spot finding prior to single molecule imaging and for implementations where the single molecules to be analysed are not organised within the spatially addressable microarray spots, a series of images of the surface can be taken by x-y translation of the slide. A super-wide field image is then composed by stitching each of the images together. This process can be automated to form a high throughput system, utilising computer software to control the process.

### Real-time sequencing

Where real-time sequencing is carried out, the translation of sample with respect to CCD camera may be too slow to detect each molecular event. Therefore a method for collecting single molecule data on a surface by taking images simultaneously with an array of CCD chips can be applied. Alternatively, the sequencing steps can be controlled by photo-clocking as described above.

### Beyong the Diffraction limit of light

By conventional means the diffraction limit of light does not allow molecules that are closer than λ/2 to be distinguished as separate point sources of light. Scanning methods such as the Near-filed Scanning Optical Mircoscope (NSOM) are too slow for the purposes of the present invention. However there are several methods under development that in the future should be able to offer resolution beyond the diffraction lumit of light (e.g. WO03/016781). Also recently by mathematical processing it has been demonstrated that by taking several exposures and analyzing photobleaching characteristics it is possible to obtain resolution far-beyond the diffraction limit of light (Yildaz *et* a/). This would be required for the analysis of individual molecules within DNA colonies/clusters as individual strands may optimally only be separated by around 130nm (typical amplification lengths are 400base pairs.)

The sequencing methods of this invention can also be implemented using quite different detection schemes which are not constrained by the limits of optical physics. At the single molecule level, nanoparticles can be detected by electron microscopies, scanning probe microscopies and if the nanoparticles are magenetic, they can be detected by magnetic detecter heads.

### Image analysis and algorithms for sequencing

Metamorph, Cytoscout, and several other commercial software offer facilities for analysis and counting of molecules. Molecules of each fluorescent wavelength can be analysed, to provide information of which nucleotide is incorporated. An algorithm is provided for compiling the sequence.

### System and Kits

The invention is readily automated, both for small-scale operation and large-scale operation. One aspect of the invention is a kit for sequencing comprising, a polymerizing agent, special nucleotides and optionally labels, antifade comprising antioxidants and chips. The invention also includes systems and apparatus for carrying out sequencing automatically according the reagents and instruments described in this document.

The invention is described in reference to the following figures:
Figure 1 illustrates sequencing by synthesis on arrays
Figure 2 is a schematic and images of capture combed lambda DNA polymers. Sequencing by synthesis can be initiated at nicked sites using strand displacement competent polymerases. The genomic DNA is fragmented in fragemtns about 200kb in length. These are captured on a microarray and different fragments of the genome are sorted to different spots on the array. The captured molecules are combed on the surface so that the sequence becomes linearly displayed. Individual combed DNA molecules can be seen using a 100X objective. The whole genome can be covered on an about 20,000 spot microarray. Nickase activity is used to create nicks and initiate strand displaceement synthesis. Alternatively gaps can be made by T7 exonucelase and synthesis can be done with non-strand dispacement competent polymerases (see Ramanathan et al Anal Biochem. 2004 Jul 15;330(2):227-4). The incorporation of nucleotides can be monitored at multiple resolvable sites.
Figure 3 shows a β-Thalasemia microarray onto which wild-type amplicons are added and primer extension conducted by Thermosequenase utilising ssbiotin dNTPs labelled with strepatavidin Quantum Dots. The array image is of the incorporation of dCTP labelled with Qdot 565nm detected using a Genepix 4100A. The enlarged view (60X 1.45NA oil objective) shows individual Quantum dots labelling single molecules localised within the microarray spot. The incorporation is specific to the microarray spot.
Figure 4 is a schematic of a single stepwise sequencing cycle, using the NH₂-ddNTP system (A) and the alpha-s-ddNTP system (B).
Figure 5 shows a modified dNTP in which a quencher is attached to the gamma phosphate and a removable label with dual reporter and blocker function is attached to either the base or the 3' position on the sugar. It is shown that upon incorporation the quencher is released on the pyrophosphate leaving moiety, leaving the fluorescent label free to fluoresce and be detected. If the removable label is removed due to the cleavage of a photolabile group then a closed system can be implemented in which each step is iterated after illumination at the wavelength suitable for cleavage of photolabile bond.
Figure 6 shows four groups of chemically synthesized randomised oligonucleotides for ligation synthesis in the 5'-3' direction. Instead of randomization, universal nucleotides can be used. The PN cleavable bond is indicated.
Figure 7 illustrates the sequencing by ligation scheme in the 5'-3' direction.
Sequencing by ligation can also be implemented in the 3'-5' direction by using 5' phoshphorylated free ends on the array and a ligating oligonucleotide bearing a ribonucleotide cleavage system.
Figure 8 shows the cleavage reaction of a PN oligonucleotide that has been ligated to a primer(A) and the cleavage reaction of a ribonucleotide containing oligo that has been ligated to a primer (B).
Figure 9 is a schematic which shows the implementation of a Quenched ligation sequencing scheme, in which two cleavage systems are used to sequence. The first system removes the quencher only when duplex has formed and the second removes the fluorophore and the non-sequenced nucleotides after detection. This single cycle can be repeated cyclically.
Figure 10 illustrates the synthesis of an artificial stem loop and the initiation of ligation based sequencing by synthesis The asymmetric phosphoramidite doubler is attached via a linker to the glass surface and then each arm is deprotected separately to synthesize two oligos that can nnela together to form a stem loop. The sequencing template can then be ligated to the recessed end and chain extension can be inititiated. The advantage of this kind of structure is that even after harsh treatment such as alkali or acid the template is abel to renature again quickly with the primer to continue synthesis. Note that this structure can also be designed for ligation in the opposite direction and polymerase based nucleotide extension.
Figure 11 shows an algorithm for control of single molecule microarray imaging and the detection of sequencing signals and the conversion into base calls. The scheme provides provision to eliminate errors and to provide confidence scores. * This compares signals from different wavelengths from each pixel, takes into account marking from the error boxes and provides a running sequence for all molecules and indicates confidence levels. The running sequence for all molecules in the spot are then compared to detect heterozygosity, determine confidence levels and provide the sequence genotype.

The invention will now be described in the following non-limiting examples.

### Examples

It should be borne in mind that the following examples can be further optimised and the composition and concentrations of reagents used can be adjusted by those skilled in the art. Additonal components may be added as known in the art and as exemplified in the patents and publications referenced in this document. As many of the required procedures are standard molecular biology procedures that lab manual, Sambrook and Russell, Molecular Cloning A laboratory Manual, CSL Press (www.Molecular Cloning.com) can be consulted. Also Eckstein, editor, Oligonucleotides and Analogues: A Practical Approach (IRL Press, Oxford, 1991) and M. J. Gait (ed.), 1984, Oligonucleotide Synthesis; B. D. Hames & S. J. Higgins (eds.) can be consulted for DNA ysnthesis. The following two handbooks provide useful practical information: Handbook of Fluorescent Probes (Molecular Probes, www.probes.com); Handbook of Optical Filters for Fluorescence Microscopy (www.chroma.com)Other useful practical information can be found in Quake et al US20020164629.

There is a need to ensure that the reagents used are as pure as possible. This is particularly the case for nucleotides and oligonucleotides used in the invention. The oligonucleotides should be immobilized on a polymer matrix or by long linkers, for example around 100 atoms or five C18 spacers give good results.

### Primer extension with NH2-dNTPs

A 5'cy3-labeled primer primer (1.6 pmol) is annealed to the DNA template (2.4 pmol) in 20 mM MgCl2 and 50 mM NaOAc. A typical extension reaction (10 µl) contains 0.01 µM primer-template duplex, 45 mM Tris pH 9.5, 10 mM DTT, 20 mM MgCl2, 4 mM NH2-dNTPs and 5 U Klenow (exo-) polymerase (New England Biolabs). After incubation at 37°C for 1 h, 30 µl of TE (10 mM Tris, 1 mM EDTA, pH 8) is added and the mixture is purified using a Sephadex G50 column. The resultant extension product is analysed on a TBE/ Urea PAGE gel. A 15-20% gel is used for extension products ranging between 20 an 70nucelotides. When the extension is done on an array, the solution is washed off before imaging. In this case the primer is not labeled, the NH2-dNTP is labeled (Fidelity systems, USA).

### Acid cleavage of PN bond

2 µl of 1% acetic acid (HOAc) is added to each of the Sephadex purified extension products (δ µl) (see above), followed by incubation at 40°C for 30 min. Deionized water (100 µl) is added to each sample and the diluted solutions are *dried in vacuo* at room temperature. The single nucleotide cleavage can be checked on a TBE/Urea gel alongside the extended product. When the reaction is done on an array a solution of 1-10% acetic acid is passed over the array at room temperature or at a temperature up to at 40°C. Incubation may be between 30 seconds and 30 minutes.

### Enzymatic ligation

The enzymatic ligation conditions are given for the following enzymes: T4 DNA ligase, *E. coli* (NAD dependent) DNA ligase, and *Taq* DNA ligase. The standard T4 DNA ligation buffer consists of the following: 50 mM Tris-HCl (pH 7.8), 10 mM MgCl2, 10 mM DTT, 1 mM ATP, 50 µg/ml BSA, 100 mM NaCl, 0.1% TX-100 and 2.0 U/µl T4 DNA ligase (New England Biolabs). *E. coli* DNA ligase buffer consists of 40 mM Tris-HCl (pH 8.0), 10 mM MgCl2, 5 mM DTT, 0.5 mM NADH, 50 µg/ml BSA, 0.1% TX-100, and 0.025 U/µl *E. coli* DNA ligase (Amersham). *Taq* DNA ligation buffer consists of the following: 20 mM Tris-HCl (pH 7.6), 25.mM potassium acetate, 10 mM magnesium acetate, 10 mM DTT, 1 mM NADH, 50 µg/ml BSA, 0.1% Triton X-100, 10% PEG, 100 mM NaCl, and 1.0 U/µl *Taq* DNA ligase (New England Biolabs). For ligating 8-mer and 9-mers, T4 and E. *coli* DNA ligase reactions are performed at 30°C, and *Taq* DNA ligase reactions at 37 to 40°C left overnight or as short as 2 hrs.

The concentration of oligonucleotides should be at least, 50fmol in a 50µl reaction volume.

This protocol is appropriate for ligation of ligating oligonucletides containing different modification, e.g. PN modification, phosphorothioate modification, RNA modification, LNA modification, nitropyrrole modification (universal base which enhances specificity).

### Thermal cycling for ligation reactions

To enhance specificity and the rate of ligation the ligation reaction can be thermally cycled using thermostable DNA ligases such as Tth, Taq, Pfu DNA ligase in therir appropriate buffers. The following is a typical cycling scheme:
2 mins at 40-54 degrees
1 minute at 65 degrees
1 minute at 94-99 degrees
Repeat up to 20 times

When reactions are done a on a glass slide directly in contact with heated plates then the following cycles can be used
37-54 degrees 10-30 seconds
65 degrees 10-3 0 seconds
94-99 degrees 10-30 seconds
repeat up to 20x

### Chemical Ligation of 3' phosphoryl with 5' phosphoryl terminated oligonucleotides

The chemical ligation reaction uses freshly dissolved 1-ethyl-3-(3-dimethylaminopropyl)carbodiimide hydrochloride (EDC, Pierce Biochemicals) (2 M in H2O for 10Å∼ stock) to generate a pyrophosphate bond between a 5'-phosphate moiety of the target and a 3'-phosphate moiety on the complementary array oligonucleotide. The chemical ligation conditions are as follows: 50 mM 2-[*N*-morpholino]ethanesulfonic acid (MES) (pH 6.0 with KOH), 10 mM MgCl2, 0.001% SDS, 200 mM EDC, 50 mM imidazole (pH 6.0 with HCl) and 3.0-4.0 M TMACl (Sigma) for 14 hr at 30°C. 3-4 M tetramethylammonium chloride (TMACl) can be used in the ligation buffer to help normalize the intensities of A/T-rich and G/C-rich probes

Ligation can be enhanced by addition of the intercalator proflavin and PEG-400.

### Chemical Ligation of 5'thiophisphate oligonucleotide with 3' OH oligonucleotide

The template directed chemical ligation of 5' phosphorothioate with 3' OH reactions is carried out in a total volume of 100 ul in a buffer containing 50 mM MES-triethylamine (pH 6.0), 20 mM MgCl2 and 10 uM of ligating oliogs and 12 uM of template. The reaction mixture is heated at 90[deg.] C. for two minutes and allowed to cool at room temperature for 2 hours. Later the reaction mixture is left on an ice bath for 1 hour and then added freshly prepared 200 mM of water-soluble diethylaminopropyl ethyl carbodiimide hydrochloride (EDC) solution. The reaction mixture is left at 4[deg.] C. for 24 hours. After 24 hours, the reaction mixture is made up to 1.0 ml with sterile water. The samples are then desalted, lyophilized and redissolved in 100 ul of in water.

### Cleavage of a photolabile linker

A photocleavable 2-nitrobenzyl linker at 3' end can be used as a photoreversible linker for a blocker and/or label. Photocleavable is achieved by UV lighta at 355nm at 1.5W/cm2, 50mJ/pulse. One pulse is for 7ns and this is repeated for a total of 10 sec.

### Cleavage of ribonucleotide linkage

This is cleaved by incubation with alkali, for example warm NaOH at 0.1N concentration.

### Exonuclease digestion

25-200U/slide of exonuclease III in 66mM Tris-HCl, 10mM MgSO4, pH8.0 in a volume of 40ul was incubated for 15 minutes (or 30 minutes or overnight) at room temperature (or 37 C).

### Preparation of artificial stem loop array for sequencing

An array of artifical stem-loop oligonculeitdes attached to a glass surface via a ling linker and an asymmetric doubler reagent can be made.This can be done by a an Agilent array synthesizer or by using as an ABI 394 DNA synthesizer as and physical masking described in Southern et al (Nucleic Acid Research 1994). It can also be done by synthesizing oligos off the chip and spotting a microarray (eg Amersham Lucidea spotter)

The oligonucletides in this case must start with a terminal amino modification with which they can be attached to an activated surface (e.g an epoxysilane surface or several types of surfaces available commercially). An array can also be made by spreading a random array by spotting between 1 and 0.1µM of aminated oligos onto an aminosilane slide in DMSO.

Using conventional routines on (e.g as recommended by Glen Research or as known in the art) ) for automated DNA synthesis, Add up to 5 C18 linkers linkers, add a asymmetric doubler, deprotect one arm and synthesize oligonucelotide in 3'5 direction. Then deprotect second arm and synthesize partly complennary oligo in the 5' 3 direction with a 3' non complementary section. Anneal by heating array to 95 and cool in buffer containing monovalent and or divalent salts, to generate sticky end. Ligate target DNA to recessed strand of sticky end. Prime synthesis with other strand.

### Sequencing by synthesis with DNA polymerase on glass microarray surfaces

A glass microarray contains oligos contain a series of oligonucleotides for probing B-thalassemia mutations (purchased from Asper Biotech, Estonia).

### Fragmentation of the templates

PCR amplification amplicons from regions of intereste for B-thalasemia (purchased from Asper Biotec, Estonia) were fragmented and unincorporated dNTPs functionally inactivated by shrimp Alkaline Phosphatase (sAP, 1U/Rx Amersham Biosciences Inc., Piscataway, NJ, USA) and Uracil N-Glycosylase (UNG, 1U/Rx, Epicentre Technologies, Madison, WI, USA) treatment at 37°C for 1 hour. Alternatively, whole genomic sample or sonicated genomic sample or restricted genomic sample could be used as template.

### Arrayed Primer Extension reactions (APEX)

Wash arrayed slides 1x in 95°C milliQ water for 2 minutes, 1x with 100mM NaOH for 5 minutes and in 95°C milliQ water 2 times for 2 minutes each. A 40 µl APEX reaction volume in 1x ThermoSequenase reaction buffer was prepared as follows: 15ul UNG/sAP treated heat denatured PCR fragments, fluorescently labelled ddNTP mix (Fluorescein-12 ddGTP, Cy3 ddCTP, Texas Red ddATP, Cy5 ddUTP - each at 1.25uM, alternatively alpha-S-dideoxynucleotides have also been used), 3U/Rx ThermoSequenase DNA polymerase, Amersham Biosciences Inc., Piscataway, NJ, USA (alternatively Vent exo- DNa Polymerase has also been used), milliQ H2O to 40ul. Apply reaction onto a pre-warmed arrayed slide. Apply a cover slip, and incubate at 58°C for 10 minutes.

After dideoxy incorporation and chain termination, the slide was scanned to observe primer extension. Alternatively, the slide was observed in a microscope under epifluorescence conditions.

### Exonuclease III treatment of extended primer reactions.

Exonuclease III is known for its ability to remove 3' blocks from DNA synthesis primers in damaged *E. coli* and restore normal 3' hydroxyl termini for subsequent DNA synthesis (Demple B et al, PNAS, 83, 7731-7735, 1986). Magnesium concentration is critical for the activity of exonuclease III, 10mM has been reported (Werner, Los Alamos, 2002). However in the quoted Demple paper the concentration of magnesium was kept to 5mM (MgCl2) in 50mM Tris-HCl, pH 7.5/ 5mM 2-mercaptoethanol/50mM NaCl. The slide was treated to an exonuclease digestion to remove the blocking group on the dideoxy nucleotide. 100U/slide (or 50 or 200 U) of exonuclease III in 66mM Tris-HCl, 10mM MgSO4, pH8.0 in a volume of 40ul was incubated for 15 minutes (or 30 minutes or overnight) at room temperature (or 37 C). After this time the slide was washed in 95 C milliQ water for 2 minutes, then in 0.3% Alconox for 3 minutes and then twice in 95 C milliQ water for 2 minutes each. The removal of fluorescent signal was detected either by scanning the slide or by placing the slide on an epifluorescent microscope or, when performing in solution reactions, by examining the DNA band pattern after polyacrilamide gel electrophoresis.

Some reactions were performed in the presence of Quantum Dot streptavidin nucleotide conjugates (565 C and 655G, Quantum Dot Corporation, USA). This was incorporated into the primer and detected under epifluorescence microscopy using a droplet of SlowFade® Light Antifade Reagent (Molecular Probes, Eugene, OR, USA) between the slide and a coverslip and the appropriate miscroscope settings. A reducing reaction in 10mM TCEP (or 1 or 5 or 25mM) for 10' minutes was followed by a further microscope examination to detect removal of the Quantum Dots.

The streptavidin Quantum Dots were conjugated to ss-Biotin dNTPS(Perkin Elmer) in Quanatum Dot buffer for several days at 4 degrees C, followed by 3X ultracentrifugation and removal of supernatant at 100,000rpm on a Beckman Optima. The Qdots-dNTPs were qunatitated with nanodrop spectrophotomer (Nanodrop corp, USA). Alternatively the incubation can be carried out at 45 degrees C for 1 hour.

**THE FOLLOWING POLYUMERASE REACION BUFFER CAN ALSO BE USED WHEN SS LINKAGE IS USED: (20 MM TRIS-HCL, PH 8.8, 10 MM MGCL2, 50 MM KCL, 0.5 MG/ML BSA, 0.01% TRITON X-100).**

### Primer extension with ss-biotin nucleotides and labeling with Quantum Dots

After primer extension, as described above but by using ss-biotin dNTPs which have not been linked. Then the Quantum dots are incubated with the array at 45 °C in Quantum Dot buffer at a concentration between 4nM and 20nM.

### REFERENCES

Asanov AN, Wilson WW, Oldham PB. Regenerable biosensor platform: a total internal reflection fluorescence cell with electrochemical control. Anal Chem. 1998 Mar 15;70(6):1156-63

Bai X, Li Z, Jockusch S, Turro NJ, Ju J. Photocleavage of a 2-nitrobenzyl linker bridging a fluorophore to the 5' end of DNA. Proc Natl Acad Sci USA. 2003 Jan 21

Braslavsky I, Hebert B, Kartalov E, Quake SR. Proc Natl Acad Sci U S A. 100:3960-4. Sequence information can be obtained from single DNA molecules. (2003)

Brakmann S, Lobermann S.A. further step towards single-molecule sequencing: Escherichia coli exonuclease III degrades DNA that is fluorescently labeled at each base pair. Angew Chem Int Ed Engl. 2002 Sep 2;41(17):3215-7.

Brautigam,C.A. and Steitz,T.A. (1998) Structural principles for the inhibition of the 3'-5' exonuclease activity of Escherichia coli DNA polymerase I by phosphorothioates. J. Mol. Biol., 277,363-37

Brenner S, Johnson M, Bridgham J, Golda G, Lloyd DH, Johnson D, Luo S, McCurdy S, Foy M, Ewan M, Roth R, George D, Eletr S, Albrecht G, Vermaas E, Williams SR, Moon K, Burcham T, Pallas M, DuBridge RB, Kirchner J, Fearon K, Mao J, Corcoran K. Gene expression analysis by massively parallel signature sequencing (MPSS) on microbead arrays. Nat Biotechnol. 2000 Jun;18(6):630-4

Case-Green, S, Pritchard, C, Southern, EM 1999. Use of oligonucleotie arrays in enxymatic assays: assay optimisation. In : Schena, M. (ed.), DNA-microarrays: A practical approach. Oxford University Press, Oxford, UK pp.61-76

Cao H, , Wang J, Tegenfeldt P, Austin RH, Chen E, Wei W and Chou SY Fabrication of 10nm Enclosed Nanofluidic Channels (2002) Applied Physics Letters, Vol. 81, No. 1, pp174

Csaki A, Moller R, Straube W, Kohler JM, Fritzsche W. DNA monolayer on gold substrates characterized by nanoparticle labeling and scanning force microscopy. Nucleic Acids Res. 2001 Aug 15;29(16):E81.

Czlapinski, J. L.; Sheppard, T. L.; J. Am. Chem. Soc.; (Communication); 2001; 123(35); 8618-8619 Nucleic Acid Template-Directed Assembly of Metallosalen-DNA Conjugates

Delaney JC, Henderson PT, Helquist SA, Morales JC, Essigmann JM, Kool ET. High-fidelity in vivo replication of DNA base shape mimics without Watson-Crick hydrogen bonds. Proc Natl Acad Sci USA. 2003 Apr 15;100(δ):4469-73. Epub 2003 Apr 03.

Dawson E, et al . (2002). A first-generation linkage disequilibrium map of human chromosome 22. Nature 418: 544-8

D. Di Giusto and G. C. King Single base extension (SBE) with proofreading polymerases and phosphorothioate primers: improved fidelity in single-substrate assays Nucleic Acids Res., February 1, 2003; 31(3): e7 - 7.)

Dimalanta ET, Lim A, Runnheim R, Lamers C, Churas C, Forrest DK, de Pablo JJ, Graham MD, Coppersmith SN, Goldstein S, Schwartz DC. A microfluidic system for large DNA molecule arrays.Anal Chem. 2004 Sep 15;76(18):5293-301.

Di Giusto DA, King GC. Strong positional preference in the interaction of LNA oligonucleotides with DNA polymerase and proofreading exonuclease activities: implications for genotyping assays. Nucleic Acids Res. 2004 Feb 18;32(3):e32. Eckardt LH, Naumann K, Pankau WM, Rein M, Schweitzer M, Windhab N, von Kiedrowski G.DNA nanotechnology: Chemical copying of connectivity. Nature. 2002 Nov 21;420(6913):286

Ecker DJ, Vickers TA, Hanecak R, Driver V, Anderson K. Rational screening of oligonucleotide combinatorial libraries for drug discovery. Nucleic Acids Res. 1993 Apr 25;21(8):1853-6).

Eckstein F. Nucleoside phosphorothioates. Annu Rev Biochem. 1985;54:367-402. Review.

Egeland RD, Marken F, Southern EM. An electrochemical redox couple activitated by microelectrodes for confined chemical patterning of surfaces. Anal Chem. 2002 Apr 1;74(7):1590-6.

Finzi, L and Gelles J Science 267:378 (1995)

Henner WD, Grunberg SM, Haseltine WA. Enzyme action at 3' termini of ionizing radiation-induced DNA strand breaks. J Biol Chem. 1983 Dec 25;258(24):15198-205.

Lee LG, Connell CR, Woo SL, Cheng RD, McArdle BF, Fuller CW, Halloran ND, Wilson RK.DNA sequencing with dye-labeled terminators and T7 DNA polymerase: effect of dyes and dNTPs on incorporation of dye-terminators and probability analysis of termination fragments. Nucleic Acids Res. 1992 May 25;20(10):2471-83

Ramanathan A, Huff EJ, Lamers CC, Potamousis KD, Forrest DK, Schwartz DC. An integrative approach for the optical sequencing of single DNA molecules. Anal Biochem. 2004 Jul 15;330(2):227-41

Sauer S, Lechner D, Berlin K, Lehrach H, Escary JL, Fox N, Gut IG.A novel procedure for efficient genotyping of single nucleotide polymorphisms.Nucleic Acids Res. 2000 Mar 1;28(5):E13.

Shendure J, Mitra RD, Varma C, Church GM. Advanced sequencing technologies: methods and goals. Nat Rev Genet. 2004 May;5(5):335-44.

Hermanson GT, Bioconjugate Techniques, Academic Press 1996

Heaton PA, Eckstein F.Diastereomeric specificity of 2',3'-cyclic nucleotide 3'-phosphodiesterase. Nucleic Acids Res. 1996 Mar 1;24(5):850-3.

Fodor S.P.A et al Science 251:767 (1991)

Gao X, LeProust E, Zhang H, Srivannavit O, Gulari E, Yu P, Nishiguchi C, Xiang Q, Zhou X. A flexible light-directed DNA chip synthesis gated by deprotection using solution photogenerated acids.Nucleic Acids Res. 2001 Nov 15;29(22):4744-50.

Xiaolian Gao, Peilin Yu, Eric LeProust, Laetitia Sonigo, Jean Phllippe Pellois and Hua Zhang Oligonucelotide synthesis using solution Photogenerated acids. J. Am Chem. Soc 120: 12698-12699 (1998)

Gordon MP, Ha T, Selvin PR. Single-molecule high-resolution imaging with photobleaching. Proc Natl Acad Sci USA. 2004 Apr 27;101(17):6462-5. Epub 2004 Apr 19.

Gueroui Z, Place C, Freyssingeas E, Berge B. Observation by fluorescence microscopy of transcription on single combed DNA. Proc Natl Acad Sci USA. 2002 Apr 30;99(9):6005-10.

Gunderson KL, Huang XC, Morris MS, Lipshutz RJ, Lockhart DJ, Chee MS. Mutation detection by ligation to complete n-mer DNA arrays. Genome Res. 1998 Nov;8(11):1142-53.

Hamad-Schifferli K, Schwartz JJ, Santos AT, Zhang S, Jacobson JM. Remote electronic control of DNA hybridization through inductive coupling to an attached metal nanocrystal antenna. Nature. 2002 Jan 10;415(6868):152-5

He K, Porter KW, Hasan A, Briley and JD, Shaw BR Synthesis of 5-substituted 2'-deoxycytidine 5'-(alpha-P-borano)triphosphates, their incorporation into DNA and effects on exonuclease. Nucleic Acids Res. Apr 15;27(8):1788-94 (1999)

Hegner H, et al. FEBS Letters 336(3):452 (1993)

Hesse J, Sonnleitner M, Sonnleitner A, Freudenthaler G, Jacak J, Hoglinger O, Schindler H, Schutz GJ. Single-molecule reader for high-throughput bioanalysis. Anal Chem. 2004 Oct 1;76(19):5960-4.

Howell WM, Jobs M, Brookes AJ 2002 Genome Res. Sep;12(9):1401-7.

Kartalov EP, Unger MA, Quake SRPolyelectrolyte surface interface for single-molecule fluorescence studies of DNA polymerase. Biotechniques. 2003 Mar;34(3):505-10.

Kartalov EP, Quake SR. Microfluidic device reads up to four consecutive base pairs in DNA sequencing-by-synthesis. Nucleic Acids Res. 2004 May 20;32(9):2873-9. Print 2004.

G. von Kiedrowski, A self-replicating hexadeoxynucleotide. Angew. Chem. Int. Ed. Engl. 25, 932-935 (1986).

Klostermeier D, Sears P, Wong CH, Millar DP, Williamson JR. A three-fluorophore FRET assay for high-throughput screening of small-molecule inhibitors of ribosome assembly. Nucleic Acids Res. 2004 May 17;32(9):2707-15.

Kneipp K, Kneipp H, Itzkan I, Dasari RR, Feld MS. Ultrasensitive chemical analysis by Raman spectroscopy. Chem Rev. 1999 Oct 13;99(10):2957-76.

Kricka LJ. Stains, labels and detection strategies for nucleic acids assays. Ann Clin Biochem. 2002 Mar;39(Pt2):114-29.

Lee HY, Sacho Y, Kanki T, Tanaka H, Shirakawa H, Cheon JW, Yoon JH, Kang NJ, Park JI, Kawai T. J DNA-directed magnetic network formations with ferromagnetic nanoparticles.Nanosci Nanotechnol. 2002 Dec;2(6):613-5.

Kanan MW, Rozenman MM, Sakurai K, Snyder TM, Liu DR. Reaction discovery enabled by DNA-templated synthesis and in vitro selection. Nature. 2004 Sep 30;431(7008):545-9.

Kartalov EP, Unger MA, Quake SR. Polyelectrolyte surface interface for single-molecule fluorescence studies of DNA polymerase. Biotechniques. 2003 Mar;34(3):505-10.

Kartalov EP, Quake SR.Microfluidic device reads up to four consecutive base pairs in DNA sequencing-by-synthesis.Nucleic Acids Res. 2004 May 20;32(9):2873-9.

Karimi-Busheri F, Lee J, Tomkinson AE, Weinfeld M. Repair of DNA strand gaps and nicks containing 3'-phosphate and 5'-hydroxyl termini by purified mammalian enzymes. Nucleic Acids Res. 1998 Oct 1;26(19):4395-400. Kunkel,TA., Eckstein,F., Mildvan,A.S., Koplitz,R.M. and Loeb,L.A. (1981) Deoxynucleoside [1-thio]triphosphates prevent proofreading during in vitro DNA synthesis. Proc. Natl Acad Sci. USA, 78, 6734―6738

AJ Lacey Light Microscopy in Biology, A practical Approach. OUP

Latif S, Bauer-Sardina I, Ranade K, Livak KJ, Kwok PY. Fluorescence polarization in homogeneous nucleic acid analysis II: 5'-nuclease assay. Genome Res. 2001 Mar;11(3):436-40.

Levene MJ, Korlach J, Turner SW, Foquet M, Craighead HG, Webb WW. Zero-mode waveguides for single-molecule analysis at high concentrations. Science. 2003 Jan 31;299(5607):682-6.

Li Z, Bai X, Ruparel H, Kim S, Turro NJ, Ju J. A photocleavable fluorescent nucleotide for DNA sequencing and analysis. Proc Natl Acad Sci USA. 2003 Jan 21;100(2):414-9.

Livak KJ.SNP genotyping by the 5'-nuclease reaction. Methods Mol Biol. 2003;212:129-47.

Malicka J, Gryczynski I, Fang J, Kusba J, Lakowicz JR. Increased resonance energy transfer between fluorophores bound to DNA in proximity to metallic silver particles. Anal Biochem. 2003 Apr 15;315(2):160-9.

Metzker ML, Raghavachari R, Richards S, Jacutin SE, Civitello A, Burgess K, Gibbs RA. Termination of DNA synthesis by novel 3'-modified-deoxyribonucleoside 5'-triphosphates. Nucleic Acids Res. 1994 Oct 11;22(20):4259-67.

Mitra RD, Shendure J, Olejnik J, Edyta-Krzymanska-Olejnik, Church GM. Fluorescent in situ sequencing on polymerase colonies. Anal Biochem. 2003 Sep 1;320(1):55-65.

Nakayama H, Arakaki A, Maruyama K, Takeyama H, Matsunaga T. Single-nucleotide polymorphism analysis using fluorescence resonance energy transfer between DNA-labeling fluorophore, fluorescein isothiocyanate, and DNA intercalator, POPO-3, on bacterial magnetic particles. Biotechnol Bioeng. 2003 Oct 5;84(1):96-102.

Nakao Hidenobu, Hiroshi Shiigi, Yojiro Yamamoto, Shiho Tokonami, Tsutomu Nagaoka, Shigeru Sugiyama, and Toshio Ohtani. Highly Ordered Assemblies of Au Nanoparticles Organized on DNA. Nano letters Vol 3 pp 1391―1394 2003

S.A.E. Marras, F.R Kramer, and S. Tyagi, Nucleic Acids Res., 2002, 30, E122

M.A. Osborne, C.L. Barnes, S. Balasubramanian, and D. Klenerman, "Probing DNA surface attachment and local environment using single molecule spectroscopy", J. Phys. Chem. B, 105,3120-3126 (2001)

Osborne MA, Furey WS, Klenerman D, Balasubramanian S. Single-molecule analysis of DNA immobilized on microspheres. Anal Chem. 2000 Aug 1;72(15):3678-81.

Paez JG, Lin M, Beroukhim R, Lee JC, Zhao X, Richter DJ, Gabriel S, Herman P, Sasaki H, Altshuler D, Li C, Meyerson M, Sellers WR.Genome coverage and sequence fidelity of phi29 polymerase-based multiple strand displacement whole genome amplification. Nucleic Acids Res. 2004 May 18;32(9):e71.

Pastinen T, Raitio M, Lindroos K, Tainola P, Peltonen L, Syvanen AC. A system for specific, high-throughput genotyping by allele-specific primer extension on microarrays.Genome Res. 2000 Ju1;10(7):1031-42.

Paunesku T, Rajh T, Wiederrecht G, Maser J, Vogt S, Stojicevic N, Protic M, Lai B, Oryhon J, Thurnauer M, Woloschak G. Biology of TiO2-oligonucleotide nanocomposites. Nat Mater. 2003 May;2(5):343-6.

Ronaghi M, Uhlen M, Nyren P. A sequencing method based on real-time pyrophosphate. Science. 1998 Jul 17;281(5375):363,36

Ronaghi M, Pettersson B, Uhlen M, Nyren P. PCR-introduced loop structure as primer in DNA sequencing. Biotechniques. 1998 Nov;25(5):876-8, 880-2, 884

Sanger F, Nicklen S, Coulson AR. DNA sequencing with chain-terminating inhibitors. Proc Natl Acad Sci USA. 74:5463-7 (1977)

Seo TS, Bai X, Ruparel H, Li Z, Turro NJ, Ju J. Photocleavable fluorescent nucleotides for DNA sequencing on a chip constructed by site-specific coupling chemistry. Proc Nat1 Acad Sci USA. 2004 Apr 13;101(15):5488-93. Epub 2004 Apr 02.

Shchepinov MS, Korshun VA. Design of multidye systems for FRET-based applications. Nucleosides Nucleotides Nucleic Acids. 2001 Apr-Jul;20(4-7):369-74

Shchepinov,M.S., Denissenko,M.F., Smylie,K.J., Worl,R.J., Leppin,A.L., Cantor,C.R. and Rodi,C.P. (2001) Matrix-induced fragmentation of P3'-N5'-phosphoramidate-containing DNA: high-throughput MALDI-TOF analysis of genomic sequence polymorphisms. Nucleic Acids Res., 29, 3864―3872

Smimov DA, Burdick JT, Morley M, Cheung VG.Method for manufacturing whole-genome microarrays by rolling circle amplification. Genes Chromosomes Cancer. 2004 May;40(1):72-7.

Sosnowski RG, Tu E, Butler WF, O'Connell JP, Heller MJ. Rapid determination of single base mismatch mutations in DNA hybrids by direct electric field control. Proc Natl Acad Sci USA. 1997 Feb 18;94(4):1119-23

Stryer, L. and Haugland, R.P. 1967. Energy transfer: A spectroscopic ruler. Proc. Natl. Acad. Sci. 58: 719-726

Tegenfeldt JO, Prinz C, Cao H, Huang RL, Austin RH, Chou SY, Cox EC, Sturm JC Micro- and nanofluidics for DNA analysis (2004) Anal Bioanal Chem. Vol 378, No 7, pp 1678-1692

Thaler DS, Liu S and G Tombline. Extending the chemistry that supports genetic information transfer in vivo: Phosphorothioate DNA, phosphorothioate RNA, 2'-O-methyl RNA a

Tong AK, Jockusch S, Li Z, Zhu HR, Akins DL, Turro NJ, Ju J. Triple fluorescence energy transfer in covalently trichromophore-labeled DNA. J Am Chem Soc. 2001 Dec 26;123(51):12923-4.. PNAS 93: 1352-1356 (1996)

Unger M, Kartalov E, Chiu CS, Lester HA, Quake SR. Single-molecule fluorescence observed with mercury lamp illumination. Biotechniques. 1999 Nov;27(5):1008-14.

Vyawahare S. Eyal S. Mathews KD. Quake SR. Nanometer-scale fluorescence resonance optical waveguides. Nano Letters. 4(6):1035-1039, 2004 Jun.

Verma S, Eckstein F.Modified oligonucleotides: synthesis and strategy for users. Annu Rev Biochem. 1998;67:99-134. Review.

Wolfe,JL, T. Kawate, A. Belenky, and V. Stanton Jr Synthesis and polymerase incorporation of 5'-amino-2',5'-dideoxy-5'-N-triphosphate nucleotides Nucleic Acids Res., September 1, 2002; 30(17): 3739 - 3747.

Xu Y, Karalkar NB, Kool ET. Nonenzymatic autoligation in direct three-color detection of RNA and DNA point mutations. Nat Biotechnol. 2001 Feb;19(2):148-52.

Yildiz A, Forkey JN, McKinney SA, Ha T, Goldman YE, Selvin PR. Related Articles, Links

Myosin V walks hand-over-hand: single fluorophore imaging with 1.5-nm localization. Science. 2003 Jun 27;300(5628):2061-5. Epub 2003 Jun 05

Zander, C, Enderlain, J, Keller, RA, Single Molecule Detection in Solution Wiley-VCH 2002.

The invention provides the following:
1. A method of sequencing a target polynucleotide comprising the steps of:
   (a) Carrying out template derived nucleotide synthesis utilising a labelled nucleotide;
   (b) detecting the presence or absence of said labelled nucleotide;
   (c) replacing said labelled nucleotide with an unlabelled nucleotide; and
   (d) repeating steps a) to c)
      with the proviso that if said labelled nucleotide is labelled with a label directly attached to the nucleotide, then the replacement of said labelled nucleotide comprises removal of the whole of said labelled nucleotide and replacement with an unlabelled nucleotide, and only said labelled nucleotide can be removed.
2. A method as described in item 1 wherein said target polynucleotide is attached to a solid surface.
3. A method as described in item 1 or item 2 wherein said labelled nucleotide is labelled with a fluorescent tag.
4. A method as described in item 3 wherein said fluorescent tag is attached directly to said nucleotide.
5. A method as described in item 3 or item 4 wherein said labelled nucleotide is attached to a quencher at the gamma position, and said fluorescent tag is attached at the 3' position or to the base.
6. A method as described in item 4 or item 5 wherein step (c) comprises chemically inactivating or bleaching said fluorescent tag.
7. A method as described in item 4 wherein step (c) comprises removal of said labelled nucleotide and replacement with an unlabelled nucleotide, wherein said unlabelled nucleotide is a degradation resistant nucleotide.
8. A method as described in any one of items 1 to 4 wherein said labelled nucleotide is a degradation labile nucleotide.
9. A method as described in anyone of items 1 to 3 wherein said labelled nucleotide is labelled with a nanoparticle.
10. A method as described in item 9 wherein said nanoparticle is a semiconductor nanocrystal.
11. A method as described in any one of items 3, 9 or 10 wherein said fluorescent tag or said nanoparticle are attached to said labelled nucleotide by a linkage.
12. A method as described in item 11 wherein said linkage comprises a binding pair.
13. A method as described in item 12 wherein said binding pair comprises streptavidin and biotin or an analog thereof.
14. A method as described in item 13 wherein said biotin or analogue thereof is 2-Iminobiotin or Desthiobiotin.
15. A method as described in item 12 or item 13 wherein said fluorescent tag or nanoparticle is conjugated to said streptavidin.
16. A method as described in any one of items 11 to 13 wherein said linkage comprises a cleavable bond.
17. A method as described in any one of items 12 to 16 wherein step (b) comprises incorporation of an unlabelled nucleotide adapted for the attachment of a fluorescent tag or nanoparticle; and attaching said fluorescent tag or nanoparticle to said unlabelled nucleotide.
18. A method as described in any one of items 9 to 17 wherein step (c) comprises removing the fluorescent tag or nanoparticle from said labelled nucleotide.
19. A method as described in item 18 wherein said fluroescent tag or nanoparticle is removed from said labelled nucleotide by cleaving the cleavable bond in the linkage attaching said fluorescent tag or nanoparticle to the nucleotide.
20. A method as described in item 15 wherein said linkage attachs one member of a binding pair to the nucleotide, and the other member of the binding pair is attached to said nanoparticle.
21. A method as described in item 9 wherein said linkage comprises a binding member attached by a cleavable bond to said nucleotide and the other binding member is attached to said fluorescent tag.
22. A method as described in item 21 wherein step (d) comprises removal of said fluorescent tag by cleaving said cleavable bond.
23. A method as described in anyone of items 1 to 3 wherein step (b) is carried out by means of an imaging technique utilising FRET (fluorescent resonance energy transfer).
24. A method as described in item 23 wherein said target polynucleotide is treated with a DNA intercalating dye.
25. A method as described in item 23 or item 24 wherein said labelled nucleotide is labelled with a label that acts as a FRET partner.
26. A method as described in anyone of items 23 to 25 wherein steps (a)-(c) occur simultaneously.
27. A method as described in any one of items 1 to 3 wherein said labelled nucleotide is an oligonucleotide and step (a) comprises ligating said oligonucleotide to a primer annealed to said target polynucleotide.
28. A method as described in item 27 wherein step (c) comprises contacting said oligonucleotide with a degradation agent to remove the label.
29. A method as described in item 27 or item 28 wherein said ligation forms a degradation resistant bond.
30. A method as described in any one of items 27 to 29 wherein said oligonucleotide comprises a degradation labile intranucleoside bond and step (c) comprises contacting said oligonucleotide with an agent that degrades said degradation labile intranucleoside bond.
31. A method as described in item 30 wherein said degradation labile intranucleoside bond is between the terminal nucleotide which is ligated to said primer and the adjacent nucleotide.
32. A method as described in any one of items 27 to 29 wherein the terminal nucleotide which is ligated to the primer is a deoxynucleotide, and at least the adjacent nucleotide is a ribonucleotide.
33. A method as described in item 27 wherein said oligonucleotide comprises the structure:
   Terminal nucleotide - N - nucleotide attached to a label - M - nucleotide attached to a quencher
      Wherein N and M are each independently a bond or at least one nucleotide;
      and M comprises a first degradation labile intranucleoside bond.
34. A method as described in item 33 wherein said ligation forms a degradation resistant bond.
35. A method of sequencing a target polynucleotide comprising the steps of:
   (a) Carrying out template derived nucleotide synthesis by ligating an labelled oligonucleotide to a primer annealed to said target polynucleotide, wherein said ligation form a degradation resistant bond , and wherein said oligonucleotide comprises the structure:
      Terminal nucleotide - N - nucleotide attached to a label - M - nucleotide attached to a quencher
         wherein N and M are each independently a bond or at least one nucleotide; and M comprises a first degradation labile intranucleoside bond ;
   (b) Contacting said oligonucleotide with a first degradation agent;
   (c) Detecting the presence or absence of said labelled oligonucleotide;
   (d) Contacting said oligonucleotide with a second degradation agent;and
   (e) Repeating steps (a)-(d)
36. A method as described in item 33 or item 34 wherein N comprises a second degradation labile intranucleoside bond, wherein said second degradation labile intranucleoside bond is resistant to the degrdation agent used to degrade the first degradation labile intranucleoside bond.
37. A method of sequencing a target polynucleotide comprising the steps of:
   (a) Carrying out template derived nucleotide synthesis by ligating an labelled oligonucleotide to a primer annealed to said target polynucleotide, wherein said ligation form a degradation resistant bond , and
      wherein said oligonucleotide comprises the structure:
      Terminal nucleotide - N - nucleotide attached to a label - M - nucleotide attached to a quencher
         wherein N and M are each independently a bond or at least one nucleotide; and M comprises a first degradation labile intranucleoside bond; and
         N comprises a second degradation labile intranucleoside bond, wherein said second degradation labile intranucleoside bond is resistant to the degradation agent used to degrade the first degradation labile intranucleoside bond;
   (b) Contacting said oligonucleotide with a first degradation agent;
   (c) Detecting the presence or absence of said labelled oligonucleotide;
   (d) Contacting said oligonucleotide with a second degradation agent;and
   (e) Repeating steps (a)-(d)
38. A method as described in item 27 wherein said oligonucleotide comprises the structure:
   Terminal nucleotide- N- nucleotide attached to a label - L - nucleotide attached to a quencher
      Wherein N is a bond or at least one nucleotide; and
      L comprises a number of nucleotides which together form a hairpin structure when said oligonucleotide is not annealed to said template.
39. A method as described in item 38 wherein N comprises a degradation labile intranucleoside bond.
40. A method as described in item 38 wherein said ligation forms a degradation resistant bond.
41. A method as described in any one of items 1 to 40 wherein said target polynucleotide forms part of an array.
42. A method as described in item 39 wherein step (b) comprises measuring the signal generated by a plurality of said labelled nucleotides.
43. A method as described in item 39 wherein step (b) comprises detecting the presence or absence of said labelled nucleotide for each individual polynucleotide.
44. A method as described in item 43 wherein said detection is carried out by means of single DNA molecule imaging.
45. A method as described in item 44 wherein said single DNA molecule imaging technique is fluorescence resonance energy transfer (FRET).
46. A method as described in item 45 wherein said polynucleotide is treated with a DNA intercalating dye.
47. A method as described in item 46 wherein the label on said labelled nucleotide acts as a FRET partner to said DNA intercalating dye.
48. A method of comparing two or more polynucleotide sequences comprising:
   a) differentially labelling the nucleotide sequences being compared;
   b) immobilising said nucleotide sequences on a surface;
   c) detecting the locus of each nucleotide sequence; and
   d) sequencing said polynucleotide sequences using a method as described in any of items 1 to 47.
49. A method as described in item 48, further comprising photobleaching the label prior to the sequencing of said polynucleotide sequence.
50. A method of resolving ambiguities in a polynucleotide sequence comprising:
   a) identifying an area of ambiguity in a polynucleotide sequence;
   b) designing probes for each of the suspected sequence possibilities; and
   c) utilising the primers formed to sequence said polynucleotide sequence utilising a method as described in any of items 1 to 47.
51. A method of sequencing mRNA comprising:
   a) contacting an array of probes designed to hybridise to mRNA molecules with a sample of mRNA under conditions whereby the mRNA will hybridise to said probes; and
   b) sequencing said mRNA utilising a method as described in any one of items 1 to 47.
52. A method as described in item 51 wherein said probe is designed to hybridise to the polyadenylation signal, 5' cap, 3' tail or the poly A tail.
53. A method of sequencing a target polynucleotide comprising the steps of:
   (a) treating said target polynucleotide with an intercalating dye;
   (b) extending a primer annealed to said target polynucleotide utilising a nucleotide labelled with a label which acts as a FRET partner to said DNA intercalating dye;
   (c) detecting the presence or absence of said nucleotide by means of an imaging technique that utilises FRET; and
   (d) repeating steps a-c;
      wherein steps (a) and (b) can occur in any order.
54. A method of sequencing a target polynucleotide comprising the steps of:
   (a) extending a primer annealed to said target polynucleotide utilising a labelled nucleotide wherein the label is directly attached to the nucleotide;
   (b) detecting the presence or absence of said labelled nucleotide within said extended primer;
   (c) removal of said labelled nucleotide, and replacement of said labelled nucleotide with an unlabelled degradation resistant nucleotide; and
   (d) repeating steps a-c;
      wherein the 3' end of said primer comprises at least one degradation resistant nucleotide.
55. A method of sequencing a target polynucleotide comprising the steps:
   (a) extending a primer annealed to said target polynucleotide utilising a labelled nucleotide wherein the label is attached to the nucleotide via a cleavable linkage;
   (b) detecting the presence or absence of said labelled nucleotide within said extended primer;
   (c) cleaving said label from said nucleotide; and
   (d) repeating steps a-c.
56. A method of sequencing a target polynucleotide comprising the steps of;
   (a) extending a primer annealed to said target polynucleotide using a nucleotide attached by a cleavable linkage to one member of a binding pair;
   (b) contacting said nucleotide with a label attached to the other member of a binding pair under conditions such that the two members of the binding pair bind to one another;
   (c) detecting the presence or absence or said label;
   (d) removal of said label and said binding pair by cleaving said cleavable linkage; and
   (e) repeating steps a-d.
57. A method of sequencing a target polynucleotide, comprising the steps of:
   (a) carrying out template derived polynucleotide synthesis utilising a nucleotide labelled with a FRET partner and at least one other polymerisation reaction component labelled with a FRET partner;
   (b) determining the nucleotide incorporated by detecting FRET interactions; and
   (c) repeating steps (a) and (b).

## Claims

1. A method of sequencing a target polynucleotide comprising: (a) Carrying out template derived nucleotide synthesis utilising a labelled nucleotide; (b) detecting the presence or absence of said labelled nucleotide; (c) replacing said labelled nucleotide with an unlabelled nucleotide; and (d) repeating steps a) to c); with the proviso that if said labelled nucleotide is labelled with a label directly attached to the nucleotide, then the replacement of said labelled nucleotide comprises removal of the whole of said labelled nucleotide and replacement with an unlabelled nucleotide, and only said labelled nucleotide can be removed; preferably said the replacement of said labelled nucleotide with an unlabelled nucleotide includes the removal of the label from said nucleotide; and optionally a washing step is conducted after each steps of (a)-(c).

2. A method of sequencing a target polynucleotide according to 1 comprising: (a) extending a primer annealed to said target polynucleotide utilizing a labelled nucleotide wherein the label is attached to the nucleotide via a cleavable linkage; (b) detecting the presence or absence of said labeled nucleotide within said extended primer; (c) cleaving said label from said nucleotide; and (d) repeating (a)-(c).

3. A method according to preceding claims where the label is a fluorescent label and optionally the fluorescently labelled nucleotides are diluted by non-fluorescently labelled nucleotides and preferably both fluorescent and non-fluorescent nucleotides are modified nucleotides; optionally the nucleotide is blocked at the 3'end and may be a dideoxynucleotide; optionally the blocking/ termination is reversible.

4. A method for sequencing according to preceding claims comprising:
incorporation of nucleotide containing quencher and label; removal of quencher simultaneously with incorporation; detection of label; optional translation of sample substrate with respect to detector; removal of label by physically induced mechanism, where removal of label correlates with resumption of next nucleotide incorporation;
preferably a quencher is attached to the gamma phosphate and a removable label is preferably attached to the base or the 3' position on the sugar, wherein the quencher is released on the pyrophosphate leaving moiety, leaving the fluorescent label free to fluoresce and be detected;
preferably a reversible blocker/terminator is provided; the removable label may have a dual reporter and blocker function and is preferably attached to the base or the 3' position on the sugar;
preferably said label and said quencher moiety interact via fluorescent resonance energy transfer (FRET), electron transfer or ground state quenching and optionally multiple FRET interactions can take place as the excitation and emission spectrum for the FRET partners overlap;
optionally FRET interactions take place between an intercalator dye/ DNA stain and the fluorescent label.
optionally the sequencing is iterated or clocked by the action of a physical signal.

5. A method according to 1 and 2 of sequencing a target polynucleotide comprising the steps of (a) extending a primer annealed to said target polynucleotide using a nucleotide attached by a removable linkage to one member of a binding pair; (b) contacting said nucleotide with a label attached to the other member of a binding pair under conditions such that the two members of the binding pair bind to one another; (c) detecting the presence or absence of said label; (d) removal of said label and said binding pair by removing said removable linkage; and (e) repeating steps a-d.

6. A method according 1 and 2 of sequencing a target polynucleotide, comprising the steps of: (a) attaching the template nucleotide to a surface to form an array; (b) combing the template nucleotide to provide a linear display of sequence along the length of said template nucleotide;(c) initiating sequencing of said template at multiple positions on the linear molecule using a method as claimed in any of preceding claims and preferably the incorporation of nucleotides can be monitored at multiple resolvable sites; optionally rolling circle amplification can be performed on a circularized target resulting in tandem copies that are combed and on which sequencing by synthesis is viewed at each position.

7. A method according to 6 where instead of attaching the template nucleotide to the surface, reactions are carried out in nanochannels/ nanofluidics.

8. A method according to the above where the molecules are trapped in solution space; optionally where said solution space comprises wells, micro or nano-scale vials, wells and structures, said structures optionally comprising droplets.

9. A method according to above where the reaction are carried out in a sequencing chip comprising microfluidic channels, optionally a system of valves and optionally designated chambers for storing reagents.

10. A method according to any one of preceding claims for sequencing polynucleotides captured on a microarray and optionally said capture is permanent.

11. A method according to any one of preceding claims for sequencing polynucleotides directly from a complex mixture.

12. A method according to any one of the preceding claims further comprising one or more elements from the group comprising:
a. acquisition of sequence information from a nick site;
b. strand displacement synthesis;
c. a homogeneous or closed system;
d. immobilisation comprising direct immobilisation of target, capture of target, immobilisation of polymerizing agent;
e. target is captured by sequence recognition comprising one or more from the group comprising the target is tailed with terminal transferase and said tail is captured, target is sequence-specifically captured according to its sequence;
f. a composition comprising single stranded binding proteins comprising E. Coli SSB and T4 gene 32 protein;
g. nucleotides which contain a reversible/removable/cleavable linker comprising 2-nitrobenzyl, disulphide bond, diol, amide, internucleoside P3'-N5'Phosphoramidate (P-N) linkage, ribonucleoside linkage, 2- Iminobiotin/Desthiobiotin-Streptavidin/streptavidin analague linkage;
h. incorporation of bases at consecutive positions which are separated from their labels by a greater than a 12 atom linker;
i. the fluorescent label is bleached, inactivated or removed after detection;
j. the said labelled nucleotide is labelled with fluorescent dyes, SERS particles, PRPs (Plasmon Resonant Particles), Quantum Dots, latex particles with embedded dye, Resonance light-scattering particles (RLP) or TiO₂ nanoparticle, an electric charge identifiable tag, electrophoretic mobility identifiable tag, raman, Surface Enhanced Resonant Raman Scattering (SERRs) tag, mass tag, nanoparticle tag;
k. enhancement of fluorescence by proximity related effects with metal and preferably said effects are plasmonic effects;
l. a labelled nucleotide which is labelled with a label that acts as a FRET partner;
m. detection which is carried out by means of an imaging technique utilising FRET (fluorescent resonance energy transfer);
n. nucleotides located within microarray spots, DNA colonies or clusters, gel or bead immobilised colonies, or RCA foci;
o. surfaces which are compatible with enzymatic reactions and have low absorption of fluorescent reaction components said surfaces comprising agarose, polyacrylamide, sol-gel, polyelectrolyte multi-layers, Bovine serume albumin/biotin/streptavidin coating or various types of polymer matrix;
p. implementation comprising one from the group comprising bulk/ensemble analysis, single molecule analysis;
q. capping of unblocked or unreacted chains;
r. incorporation of extrinsic promoters for specific RNA polymerases by transposon directed integration into sites along the polynucleotide;
s. sequencing mRNA comprising: a) contacting an array of probes designed to hybridise to mRNA molecules with a sample of mRNA under conditions whereby the mRNA will hybridise to said probes; and b) sequencing said mRNA utilising a method as claimed in any one of preceding claims, optionally said probe is designed to hybridise to the polyadenylation signal, 5'cap, 3'tail or the poly A tail;
t. comparing two or more polynucleotide sequences comprising: differentially labelling the nucleotide sequences being compared; immobilising said nucleotide sequences on a surface; detecting the locus/location of each nucleotide sequence; and sequencing said polynucleotide sequences using a method as claimed in any of preceding claims and optionally further comprising photobleaehing the label prior to the sequencing of said polynucleotide sequence, if said label is a fluorescent label.

13. A kit for carrying any of the methods of preceding claims said kit optionally comprising a polymerising agent, labelled nucleotides, optionally antifade comprising antioxidants, and chips.

14. An apparatus for carrying out any of the methods of preceding claims comprising an illumination source, an objective lens and a means for detection said apparatus optionally comprising a means for the sample and detector to be translated with respect to each other in between cycles; optionally the apparatus comprises an image splitter, preferably the objective is of high numerical aperture (NA), preferably an autofocusing capability, focus is maintained as the slide is scanned, optionally excitation via Total Internal Reflection Fluorescence microscopy (TIRF) and software control.

15. An algorithm for use in a method according to preceding claims for detection of sequencing signals and conversion into base calls; optionally said algorithm is based on decision chart of table 11.
